(19) Europäisches Patentamt
European Patent Office
Office européen des brevets

(11) Publication number: **0 106 565**
**B1**

(12) **EUROPEAN PATENT SPECIFICATION**

(45) Date of publication of patent specification: **10.09.86**

(21) Application number: **83305587.4**

(22) Date of filing: **21.09.83**

(51) Int. Cl.[4]: **C 07 C 149/40,**
C 07 C 147/14,
C 07 C 147/107, C 07 C 59/90,
C 07 C 59/64, C 07 C 51/347,
A 61 K 31/19

(54) **Leukotriene antagonists, their production, and compositions containing them.**

(30) Priority: **30.09.82 US 431944**

(43) Date of publication of application:
**25.04.84 Bulletin 84/17**

(45) Publication of the grant of the patent:
**10.09.86 Bulletin 86/37**

(84) Designated Contracting States:
**AT BE CH DE FR GB IT LI LU NL SE**

(56) References cited:
**EP-A-0 043 736**
**EP-A-0 056 172**
**DE-A-1 768 334**
**DE-A-2 102 984**
**FR-A-2 509 725**
**GB-A-2 058 785**

(73) Proprietor: **MERCK FROSST CANADA INC.**
**16711 Trans-Canada Highway**
**Kirkland Quebec (CA)**

(72) Inventor: **Belanger, Patrice C.**
**419 Promenade, d'Avignon**
**Dollard des Ormeaux Quebec, H9B 1Y4 (CA)**
Inventor: **Fortin, Rejean**
**4820 Boul. Leger**
**Montreal Quebec, H1H 1N3 (CA)**
Inventor: **Guidon, Yvan**
**409 Place Closse**
**Ile Bizard Quebec, H9C 1Y7 (CA)**
Inventor: **Rokach, Joshua**
**416 Canterbury Square Chomedey**
**Laval Quebec, H7W 4G9 (CA)**
Inventor: **Yoakim, Christiane**
**5770 Cote St. Luc Road Apt. 12**
**Montreal Quebec, H3X 2E5 (CA)**

(74) Representative: **Crampton, Keith John Allen et al**
**D YOUNG & CO 10 Staple Inn**
**London WC1V 7RD (GB)**

EP 0 106 565 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Although the chemical identity of leukotrienes was not discovered until 1979, their history actually began in Australia in 1938 when researchers discovered slow reacting substances (SRS) which caused slow contractions of smooth muscle. When their chemical identity was learned, SRS was found to be a mixture of three previously unknown substances which are related chemically to the prostaglandins and thromboxanes. They were named leukotrienes because they are made by leukocytes and have three conjugated double bonds. Leukotrienes have major effects on the smaller peripheral airways of the lungs and on the larger central passages which include the trachea and the bronchi. In the presence of an allergy trigger, like pollen or dust, leukotrienes are manufactured from fatty substances trapped in the membrane of a triggered cell. A series of reactions within the cell generates a set of different leukotrienes which are transported through the cell membrane into the blood. Then they bring about a constriction of the air passages leading to breathlessness.

The present invention provides compounds of Formula XI and XII below, and compounds that are their pharmaceutically acceptable salts. Compounds of these formulae can act as antagonists to prevent leukotriene action or as inhibitors to prevent synthesis, and can prevent or reverse leukotriene action or prevent leukotriene synthesis when administered orally. The compounds can be administered by insufflation, intravenously, rectally, topically, parenterally, including subcutaneously and intramuscarly, or nasally, and the present invention also provides pharmaceutical compositions containing the said compounds and a pharmaceutical carrier. Such compositions may be suitable for administration in the ways stated.

Because of their activity as leukotriene antagonists, compounds of the present invention are useful as anti-asthmatic, anti-allergic, and anti-inflammatory agents and are useful in treating allergic rhinitis and chronic bronchitis and for amelioration of skin diseases like psoriasis and atopic eczema. These compounds are also useful to antagonize or inhibit the properties of leukotrienes relating to cardiovascular and vascular systems.

The compounds of the present invention have the Formulae:

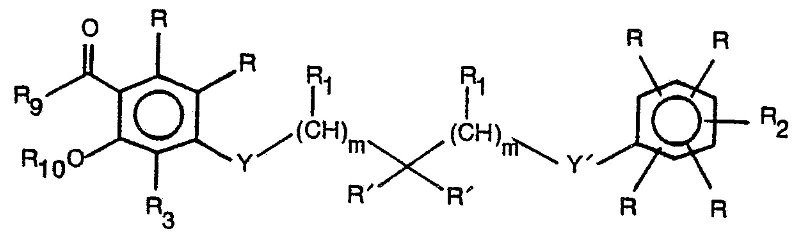

XI

and

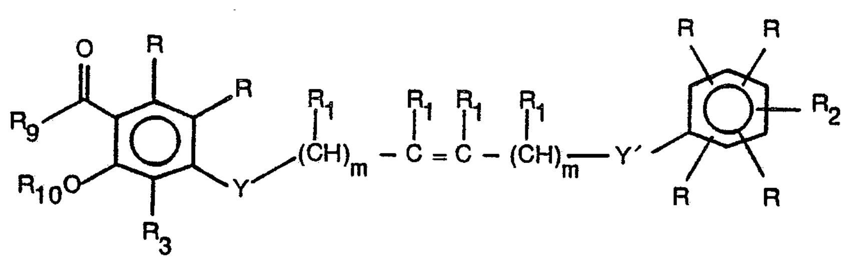

XII

wherein

each R, independently of the others, is H, OH, $C_1$—$_6$alkyl; $C_{2-6}$alkenyl; trifluoromethyl; $C_{1-6}$alkoxy; $C_{1-6}$thioalkyl; phenyl; phenyl substituted by alkyl of 1 to 3 carbon atoms or by halogen; benzyl; phen($C_{2-4}$alkyl; halogen, $COOR_4$ where $R_4$ is H or $C_{1-6}$ alkyl; CN; trifluoromethylthio; or nitro;

each R′ is independently $R_4$; $OR_4$; $COOR_4$; $N(R_4)_2$; $SR_4$; $CH_2OR_4$; CHO; or together R′ and R′ are O; $CH_2$; or

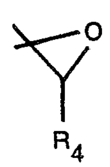

;

each of Y and Y′, which are the same as or different from one another, is oxygen, sulfur, sulfoxide, sulfone;

O
‖
$S=NR_{11}$ where $R_{11}$ is H, $C_{1-4}$alkyl, $C_{1-4}$ alkanoyl, phenylsulfonyl or tosyl;

O
‖
$N—C—R_{13}$ where $R_{13}$ is $C_{1-4}$alkyl or $C_{1-4}$ alkoxy; N—CN; $CH_2$ or C=O, but with the proviso that Y' is not oxygen unless Y is oxygen, sulfur, sulfoxide, sulfone or cyanamido and $R_2$ is

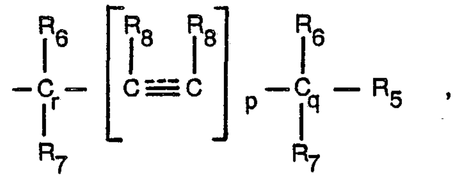

where at least one $R_7$ is OH;

each $R_1$, independently of the others, is hydrogen or $C_{1-3}$alkyl;

each m, independently of the others, is 0, 1, 2 or 3;

$R_2$ is

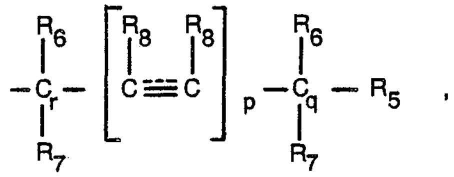

where

each $R_6$, independently of the others, is H or $C_{1-4}$alkyl;

each $R_7$, independently of the others, is H, OH, or $C_{1-4}$alkyl;

each $R_8$, independently of the others, is H or $C_{1-4}$alkyl; and is absent when a triple bond is present;

$R_5$ is $COOR_4$; $CH_2OH$; CHO; tetrazole; hydroxymethylketone; $CONHSO_2R_{14}$; CN; $CON(R_7)_2$; or $COOR_{15}$ where $R_{15}$ is:

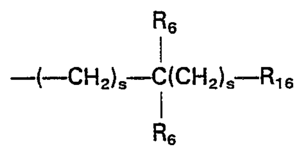

where each s is independently 0—3 and $R_{16}$ is (A) a monocyclic or bicyclic heterocyclic radical containing from 3 to 12 nuclear carbon atoms and 1 or 2 nuclear nitrogen atoms or a nuclear N and a nuclear S atom; each ring in the heterocyclic radical being formed of 5 or 6 atoms, or (B) a radical $W—R_{17}$ where W is O, S or NH and $R_{17}$ contains up to 21 carbon atoms and is a hydrocarbon radical or an acyl radical of an organic acyclic or monocyclic carboxylic acid containing not more than 1 heteroatom in the ring;

each of r and q, independently of the other, is 0 or an integer from 1 to 6, provided that r+q does not exceed 10;

p is 0 or 1;

$R_3$ is $C_{1-6}$alkyl or $C_{3-6}$alkenyl;

$R_9$ is OH, $CC_{1-6}$alkyl or $C_{1-6}$alkoxy;

$R_{10}$ is H; $R_4CO—$ or $R_4OCH_2—$;

$R_{14}$ is $C_{1-6}$alkyl, phenyl, phenyl substituted by $C_{1-3}$alkyl or alkoxy groups, halogen, hydroxy, haloalkyl, COOH, CN, formyl, $C_{1-6}$acyl or $C_{1-4}$ perfluoroalkyl; and all carbon chains in all radicals are straight or branched; or are pharmaceutically acceptable or acid-addition salts thereof.

As used herein, the term "each independently" or its equivalent is used to describe a number of possible position isomers and/or structural variations. For example, as described above, $R_2$ is

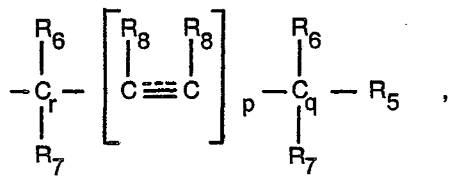

the letters r and q denoting that $C_{1-6}$alkane chains each having the $R_6$ and $R_7$ substituent groups, may or

may not be present. On each carbon atom of any such alkane chain, $R_6$ and/or $R_7$ may be the same or different. The above description therefore contemplates structures such as the following for the segments $-(CR_6R_7)_r-$ and $-(CR_6R_7)_q-$:

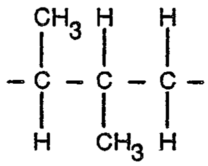

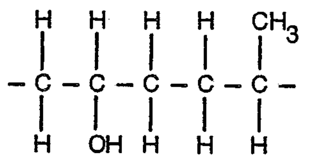

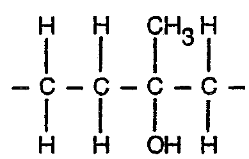

The compounds of the present invention may be prepared by several different routes. According to one method a compound of Formula I is reacted with an optionally alkyl substituted alkenyl halide of Formula II where X is halogen and each $R_6$ is independently H or $C_{1-4}$ alkyl to yield the corresponding 2-hydroxy-4-alkenyloxy-acetophenone of Formula III. The compound of Formula III is then subjected to a Claisen rearrangement to yield a 2-4-dihydroxy-3-alkenyl-acetophenone compound of Formula IV. This rearrangement occurs on heating the compound of Formula III either neat or in a high boiling solvent, such as a halogenated hydrocarbon, e.g., dichlorobenzene, at from about 160 to about 210°C. The double bond in the compound of Formula IV may then be reduced, e.g., by catalytic hydrogenation with a catalyst such as Pd/C, to yield the corresponding saturated compound of Formula V.

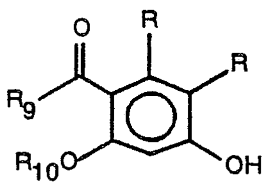

I

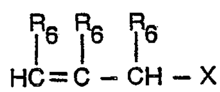

II

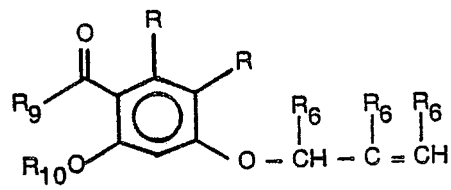

III

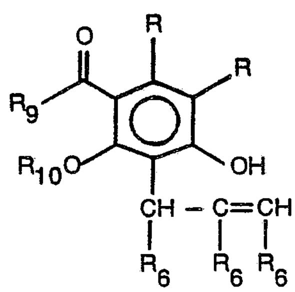

IV

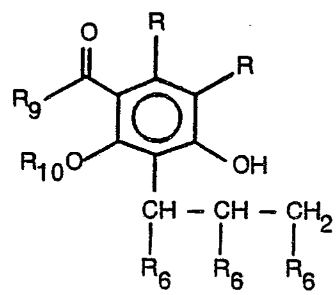

V

The compound of Formula V is then reacted with a dihaloalkane of Formula VIa or a dihaloalkene of Formula Vb wherein X, R and m have the meaning given previously, to yield a 4-(haloalkyloxy)-3-alkyl-2-hydroxyacetophenone compound of Formula VII. The reaction takes place by refluxing a mixture of the compounds of Formulae V and VIa or VIb in an inert solvent such as, for example, methylethylketone (MEK), acetone, tetrahydrofuran (THF), triglyme or dichloromethane in the presence of a base. The reflux temperature is preferably in the range of from about 60 to about 130°C. The base may be an alkali metal carbonate, for example, $Li_2CO_3$, $Na_2CO_3$ or $K_2CO_3$.

Specific examples of dihaloalkane compounds of Formula VIa are 1,3-dibromopropane, 2-methyl-1,3-dibromopropane, 2,2-dimethyl-1,3-dibromopropane, 3-chloro-2-chloromethyl-1-propene, 1,3-dibromobutane, 1,4-dibromobutane, 1,5-dibromopentane, 1,6-dibromohexane, 1,7-dibromoheptane, 1,8-dibromooctane, 1,9-dibromononane, 1,10,-dibromodecane, and 1,12-dibromododecane. A specific example of a dihaloalkene compound of Formula VIb is 1,4-bromo-2-butene.

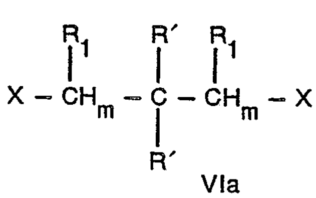

VIa

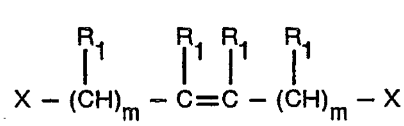

VIb

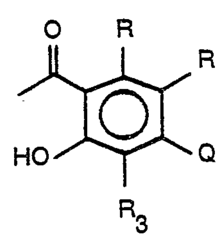

VII,

wherein Q is 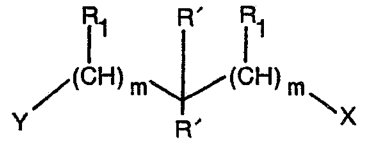 or 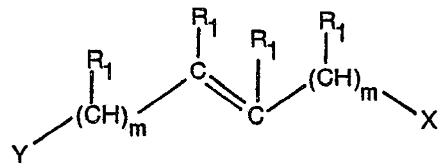

In lieu of the dihaloalkane or dihaloalkene, the compound of Formula IV or V may be reacted with an epihalohydrin, e.g., epichlorohydrin, under the same conditions to yield the 4-(2,3-epoxypropyloxy)-3-alkyl or 3-alkenyl-2-hydroxy-acetophenone compound of Formula VIII.

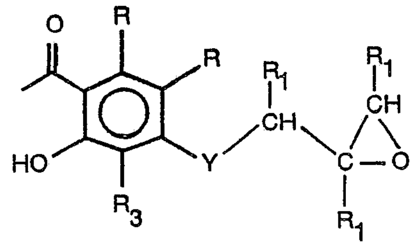 VIII

An alternative procedure is to react a compound of Formula II with a compound of Formula V to yield a 4-alkenyloxy-3-alkyl-2-hydroxy-acetophenone compound of Formula IX which is then epoxidized with an organic peracid such as, for example, m-chloroperbenzoic acid to give the compound of Formula VIII.

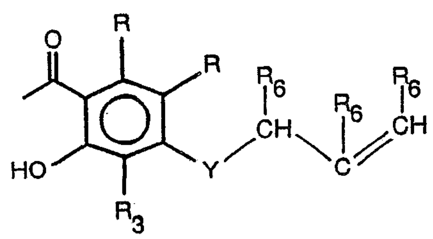 IX

The reaction of a compound of Formula VII with a compound of Formula X, under the same conditions used to react a compound of Formula V with a compound of Formula VIa or VIb, gives compounds of the Formulae XI or XII.

The reaction of a compound of Formula VIII with a compound of Formula X under the same conditions used to react a compound of Formula V with a compound of Formula VIa or VIb gives a compound of Formula XI wherein each m is 1.

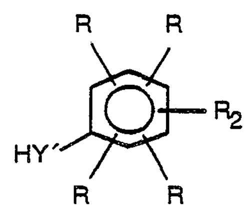

When Y' is —$CH_2$—, HY' is methyl. When Y' is $-\overset{O}{\overset{\|}{C}}-$,

HY' is a protected aldehyde, such as, for example, dithioacetal.

When Y' is methylene or carbonyl a stronger base such as, for example, lithium diisopropylamine or butyl lithium is employed in an inert solvent such as tetrahydrofuran with a suitable compound of Formula X.

A compound of Formula X is prepared by subjecting a compound of the Formula XIII

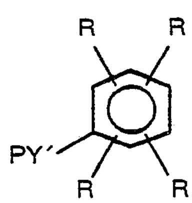 XIII

wherein P is H or a protecting group such as, for example, methyl, benzyl, *p*-nitrobenzyl or 3-(*m*-nitrophenyl)-1-phenyl-1-oxo-3-propyl to a Friedel-Crafts reaction with an acyl halide or an acid anhydride. Other compounds of Formula XI and XII are obtained by reacting a compound of Formula VII with a compound of Formula X under the same conditions used to react a compound of Formula V with a compound of Formula VIa or VIb.

Alternatively, the compounds of Formulae XI and XII may be prepared by reacting a compound of Formula X with a compound of Formula VIa or VIb or an epihalohydrin under the same conditions as described for the preparation of the compound of Formula VII to give a compound of the Formula XIVa, XIVb or XIVc respectively. The latter are then reacted under the same conditions with a compound of the Formula V to give compounds of the Formulae XI or XII.

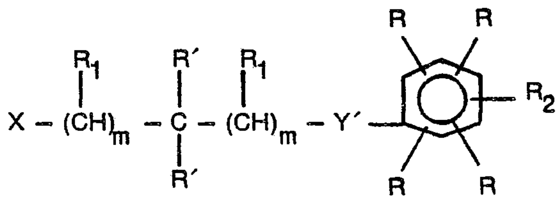

XIVa

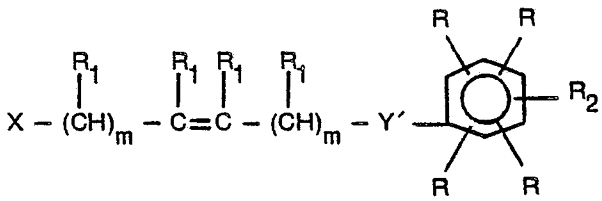

XIVb

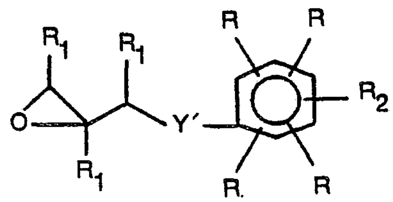

XIVc

Alternatively, but not preferably, the compounds of Formulae IV or V, X and either VIa, VIb or epihalohydrin may be reacted simultaneously under the conditions described above for reacting a compound of Formula VIa or VIb or epihalohydrin with a compound of Formula X.

Prodrug ester derivatives of the compounds of Formulae XI and XII may be prepared using conventional synthetic techniques available to the skilled person. For example, compounds of the Formula XVI:

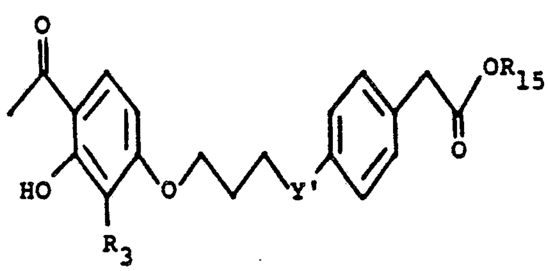

XVI

may be prepared as follows:

Method A:

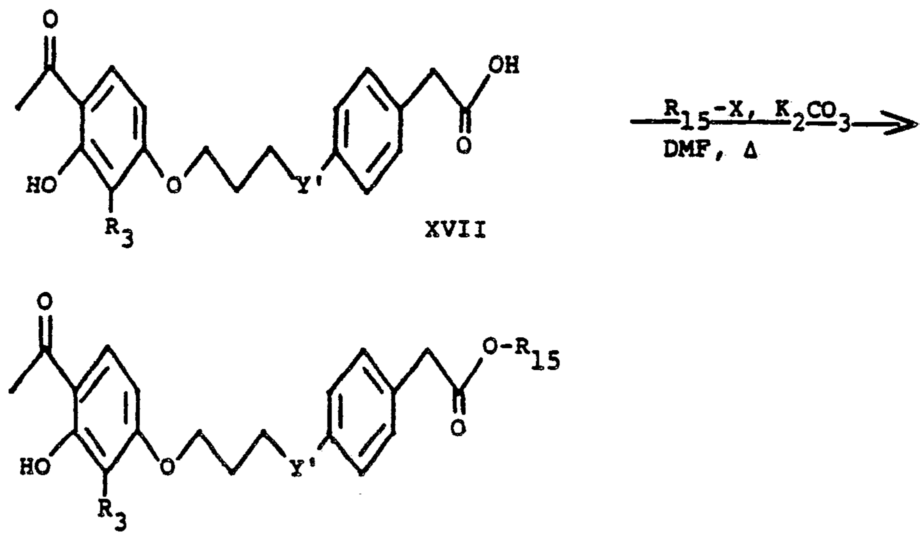


XVII

XVI

In Method A the carboxylic acid of Formula XVII is reacted in the presence of a base with an alkylhalide compound to provide the prodrug ester.

Method B:

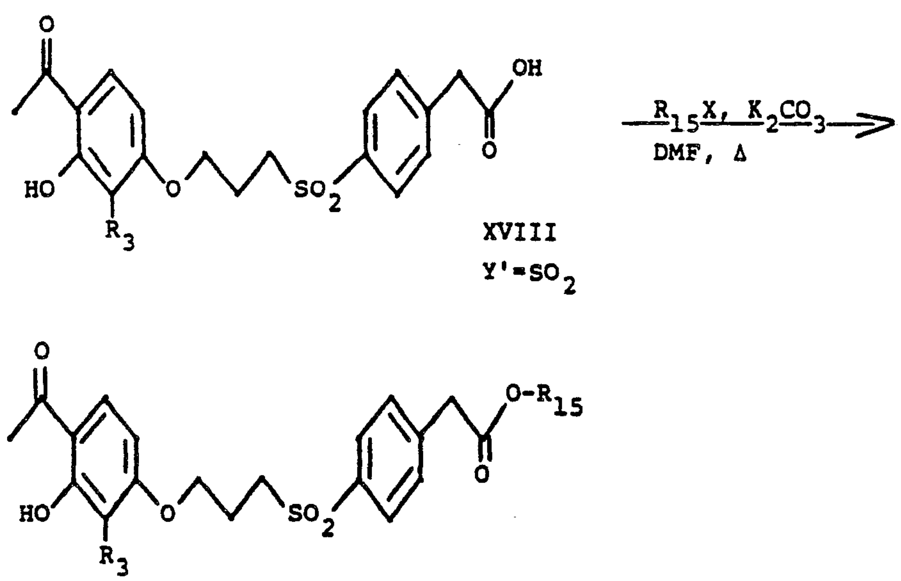


XVIII

Y'=SO2

In Method B the sulfone carboxylic acid of Formula XVIII is similarly reacted with an appropriate alkylhalide in the presence of base to provide the corresponding prodrug ester.

Method C:

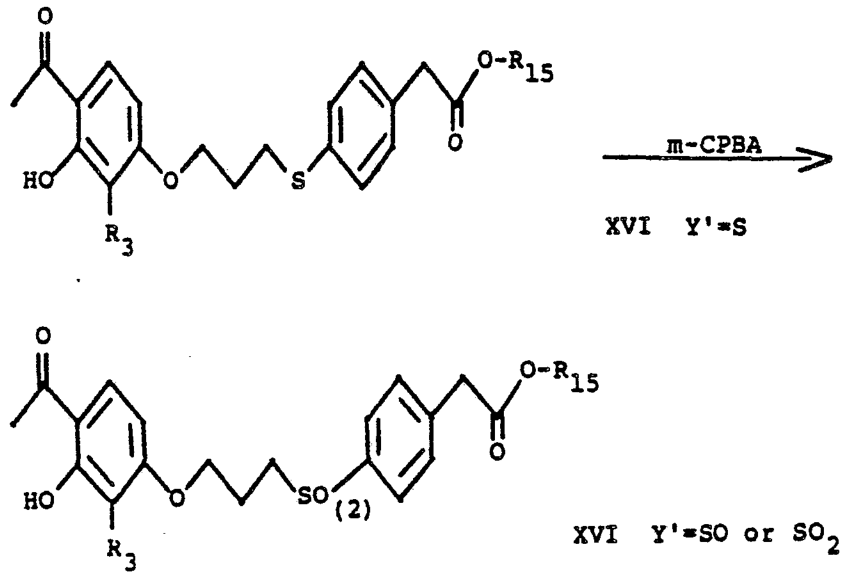


XVI Y'=S

XVI Y'=SO or $SO_2$

In Method C, the product of the Method A reaction, a prodrug ester, may be selectively oxidized to yield the sulfone or the sulfoxide compound.

In the above structures, when $R_7$=OH and $R_5$=COOH, a lactone ring may be formed which would act as a prodrug form of the hydroxy acid. For example, a dehydration reaction of the compound having the Formula XIX:

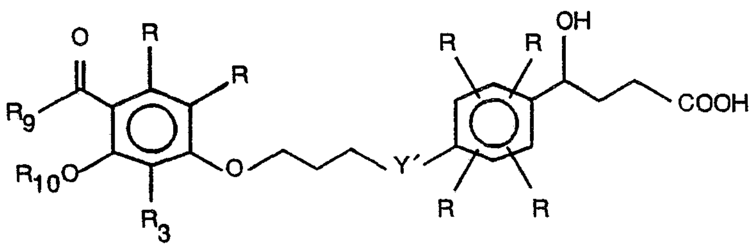

XIX

such as, by reaction with trifluoroacetic acid would yield a lactone compound having the formula:

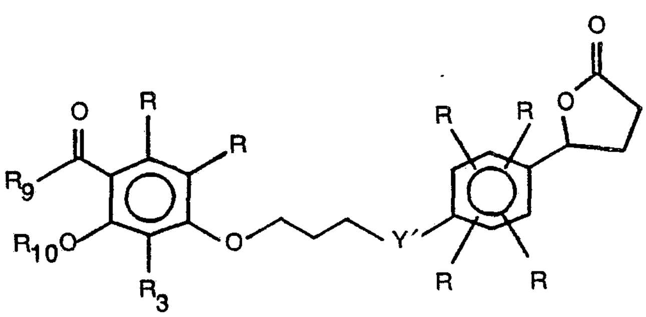

which when administered orally to a mammal would release the hydroxy acid form of the compound *in vivo*.

The magnitude of a prophylactic or therapeutic dose of a compound of Formula XI or XII will, of course, vary with the nature of the severity of the condition to be treated and with the particular compound of Formula XI or XII and its route of administration. In general, the daily dose range lies within the range of from 0.2 mg to 100 mg per kg body weight of a mammal.

The pharmaceutical compositions of the present invention comprise a compound of Formula XI or XII as an active ingredient, and may also contain a pharmaceutically acceptable carrier and optionally other therapeutic ingredients. The compositions include compositions suitable for oral, rectal, ophthalmic, pulmonary, nasal, dermal, topical or parenteral (including subcutaneous, intramuscular and intravenous)

administration, although the most suitable route in any given case will depend on the nature and severity of the conditions being treated and on the nature of the active ingredient. They may be conveniently presented in unit dosage form and prepared by any of the methods well-known in the art of pharmacy.

For use for intravenous administration, a suitable dosage range is from 0.2 to 20 mg (preferably from about 1 to about 10 mg) of a compound of Formula XI or XII per kg of body weight per day and in the case of an oral composition a suitable dosage range is, e.g. from 1 to 100 mg of a compound of Formula XI or XII per kg of body weight per day, preferably from 5 to 40 mg/kg.

For administration by inhalation, the compounds of the present invention are conveniently delivered in the form of an aerosol spray presentation from pressurized packs or a nebuliser. The preferred composition for inhalation is a powder which may be formulated as a cartridge from which the powder composition may be inhaled with the aid of a suitable device. In the case of a pressurized aerosol, the dosage unit may be determined by providing a valve to deliver a metered amount.

Pharmaceutical compositions of the present invention suitable for oral administration and by inhalation in the case of asthma therapy may be presented as discrete units such as capsules, cachets or tablets each containing a predetermined amount of the active ingredient, as a powder or granules, or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredients with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or moulding, optionally with one or more accessory ingredients. Compressed tablets may be prepared by compressing in a suitable machine, the active ingredient in a free-flowing form such as powder or granules, optionally mixed with a binder, lubricant, inert diluent, or surface active or dispersing agent. Moulded tablets may be made by moulding, in a suitable machine, a mixture of the powdered compound moistened with an inert liquid diluent. Desirably, each tablet contains from 25 mg to 500 mg of the active ingredient and each cachet or capsule contains from 25 to 500 mg of the active ingredient.

In addition to the compounds of Formulae XI and XII the pharmaceutical compositions can also contain other active ingredients, such as non-steroidal anti-inflammatory agents e.g. indomethacin, ibuprofen, sulindac and fenbufen, peripheral analgesic agents such as zomepirac and diflunisal or cyclooxygenase inhibitors. They may also contain inhibitors of the biosynthesis of the leukotrienes, e.g., those disclosed in European Patent Specification EPA—0 115 394. These pharmaceutical compositions may also contain antihistaminic agents such as Benadryl, Dramamine, Histadyl and Phenergan. These pharmaceutical compositions containing Formulae XI or XII compounds and a second active ingredient are another embodiment of the invention. The weight ratio of the Formula XI or XII compound: second active ingredient may be varied and may range from 10:1 to 1:10.

The following examples illustrate but do not limit the present invention. All temperatures are expressed in degrees Celsius.

## Example 1

4-(3-(4-Acetyl-3-hydroxy-2-propylphenoxy)propylthio) 2,3-dichlorobenzeneacetic acid

Step. A. *Preparation of 2,3-dichloro-4-methoxyacetophenone*

To a suspension of $AlCl_3$, 80 gm, in $CH_2Cl_2$, 1000 mL, was added dropwise acetylchloride, 31.6 gm. The solution was cooled at −5°C and 2,3-dichloroanisole, 70.8 gm, dissolved in $CH_2Cl_2$, 50 mL was then added. The solution was allowed to warm to room temperature and was stirred for 4 hours. The reaction mixture was poured on ice and stirred for 30 minutes. The organic layer was decanted and the aqueous layer was extracted with $CH_2Cl_2$. The combined fractions were washed with brine, dried ($Na_2SO_4$) and concentrated to a small volume. The addition of hexane caused crystallization of a solid which was filtered to yield the title compound, mp 73—77°C.

| | | | |
|---|---|---|---|
| Analysis, calculated: | C, 49.34; | H, 3.68; | Cl, 32.36. |
| Obtained: | C, 49.09; | H, 3.61; | Cl, 32.34. |

Step B: *Preparation of 2,3-dichloro-4-methoxybenzene acetic acid, methyl ester*

To a mixture of 2,3-dichloro-4-methoxyacetophenone, 58 gm, methanol, 450 mL, and 70% perchloric acid, 88 mL, cooled to 0°C, there was added thallium trinitrate trihydrate, 117 gm. The reaction mixture was allowed to warm to room temperature and was stirred for 18 hours. The mixture was poured in water, 700 mL, and was extracted twice with $CH_2Cl_2$, 500 mL. The combined organic fractions were washed successively with water, 5% $NaHCO_3$ and with water, dried ($Na_2SO_4$) and concentrated *in vacuo* to yield, after purification by chromatography on silica gel, the title compound, as an oil, readily characterized by its NMR spectrum: (ppm) ($CDCl_3$) 3.85 (3H, s), 3.73 (2H, s), 3.68 (3H, s).

Step C: *Preparation of 2,3-dichloro-4-hydroxy-benzeneacetic acid, methyl ester.*

2,3-Dichloro-4-methoxybenzeneacetic acid, methyl ester, 46 gm, was refluxed for 18 hours in concentrated HBr, 350 mL. The reaction mixture was poured in water, 1.4 L, and the solution was extracted

with EtOAc, 2 × 500 mL. The organic layers were washed with water, dried ($Na_2SO_4$) and concentrated to dryness. The residue was slurried in 20% EtOAc-hexane to yield 2,3-dichloro-4-hydroxybenzeneacetic acid, mp 189—190°C. The acid was stirred at room temperature in methanolic HCl, 100 mL, for 30 minutes. The volatiles were removed *in vacuo* and the residue was slurried in hexane, filtered and air-dried to yield the title compound, mp 105—106°C.

| | | | |
|---|---|---|---|
| Analysis calculated: | C, 45.98; | H, 3.43; | Cl, 30.16. |
| Obtained: | C, 46.10; | H, 3.65; | Cl, 30.31. |

Step D: *Preparation of 2,3-dichloro-4-dimethyl-thiocarbamoyloxybenzeneacetic acid, methyl ester*

Sodium hydride, 99%, 1.3 gm, was added to a solution of 2,3-dichloro-4-hydroxybenzeneacetic acid, methyl ester, 11.4 gm, in DMF, 100 mL. The mixture was stirred until evolution of $H_2$ gas subsided. Dimethylthiocarbamoyl chloride, 6.5 gm, was then added and the reaction mixture was stirred at room temperature for 18 hours. The reaction mixture was poured in water, 200 mL, and was extracted with ether, 500 mL. The ether layer was washed with water, dried ($Na_2SO_4$) and concentrated *in vacuo* to yield an oil that was purified by chromatography on silica gel to yield the title compound, mp 87—93°C.

| | | | | | |
|---|---|---|---|---|---|
| Analysis: calculated: | C, 44.73; | H, 4.06; | N, 4.34; | S, 9.95; | Cl, 22.00. |
| Obtained: | C, 44.44; | H, 4.15; | N, 4.05; | S, 9.05; | Cl, 21.65. |

Step E: *Preparation of 2,3-dichloro-4-dimethylcarbamoylthiobenzeneacetic acid, methyl ester*

2,3-Dichloro-4-dimethylthiocarbamoyloxybenzeneacetic acid, methyl ester, 10.7 gm, was heated under $N_2$ atmosphere at 250°C for 30 minutes. The reaction mixture was cooled to room temperature and affter a purification by chromatography on silica gel, the title compound, was obtained, mp 119—122°C.

| | | | | | |
|---|---|---|---|---|---|
| Analysis, calculated: | C, 44,73; | H, 4.06; | N, 4.34; | S, 9.95; | Cl, 22.00. |
| Obtained: | C, 44.50; | H,4.21; | N, 4.25; | S, 10.19; | Cl, 22.57. |

Step F: *Preparation of the sodium salt of 2,3-dichloro-4-mercaptobenzeneacetic acid, methyl ester*

2,3-Dichloro-4-dimethylcarbamoylthiobenzeneacetic acid, methyl ester, 3.22 gm, was refluxed in methanol, 60 mL, containing sodium methoxide, 855 mg, for 1 hour. The reaction mixture, containing about 10 millimoles of the sodium salt of the title compound, in 60 mL MeOH, was used as such in Step G.

Step G: *Preparation of 4-(3-(4-acetyl-3-hydroxy-2-propylphenoxy)propylthio-2,3-dichlorobenzeneacetic acid, methyl ester*

To 30 mL of the solution obtained in Step F was added 4-(3-bromopropoxy)-3-propyl-2-hydroxyacetophenone, 1.73 gm, and the reaction mixture was refluxed for 2 hours. It was then poured in water and extracted with EtOAc. The extract was washed with water, dried ($Na_2SO_4$) and concentrated *in vacuo*. The title compound, mp 82—85°C was obtained by chromatography of the residue on silica gel.

Step H: *Preparation of 4-(3-(4-acetyl-3-hydroxy-2-propylphenoxy)propylthio)-2,3-dichlorobenzeneacetic acid*

The ester prepared in Steg G, 964 mg, dissolved in 1N NaOH, 10 mL and in methanol, 30 mL, was refluxed for 30 minutes. The volatiles were evaporated *in vacuo.* The residue was taken up in water and the resulting solution was acidified with 20% citric acid. The mixture was extracted with EtOAc, the extracts were washed with water, dried ($NaSO_4$) and concentrated *in vacuo* to yield the title compound, mp 119—122°C.

## Example 2

4-(3-(4-Acetyl-3-hydroxy-2-propylphenoxy)-2-hydroxypropylthio)-2,3-dichlorobenzeneacetic acid

Step A: *Preparation of 4-(3-(4-acetyl-3-hydroxy-2-propylphenoxy)-2-hydroxypropylthio)-2,3-dichlorobenzeneacetic acid, methyl ester*

By following Step G of Example 1, but substituting 4-(2,3-epoxypropoxy)-3-propyl-2-hydroxyacetophenone for 4-(3-bromopropoxy)-3-propyl-2-hydroxyacetophenone, the title compound, mp 85—88°C, was obtained.

Step B: *4-(3-(4-acetyl-3-hydroxy-2-propylphenoxy)-2-hydroxypropylthio)-2,3-dichlorobenzeneacetic acid*

By following Step H of Example 1, but substituting the product of Step A of this example for the ester of Step G of Example 1, the title compound, mp 151—153°C, was obtained.

## Example 3

4-(3-(4-Acetyl-3-hydroxy-2-propylphenoxy)propylthio)-2,3-dichlorobenzeneacetic acid-S-oxide

Step A: *Preparation of 4-(3-(4-acetyl-3-hydroxy-2-propylphenoxy)propylthio)-2,3-dichlorobenzene-acetic acid-S-oxide, methyl ester*

The product obtained in Step G of Example 1, 1.4 gm, in $CH_2Cl_2$, 35 mL, was treated with m-chloroperbenzoic acid, 560 mg, for 15 minutes at 0°C. $(Ca(OH)_2$, 3 gm, was added and the resulting

suspension was stirred for 10 minutes. The solids were filtered off and the filtrate was concentrated *in vacuo* to yield the title compound, mp 153—155°C.

Step B: *Preparation of 4-(3-(4-acetyl-3-hydroxy-2-propylphenoxy)propylthio)-2,3-dichlorobenzeneacetic acid-S-oxide*

By following Step H of Example 1, but substituting the product of Step A of this example for the ester of Step G of Example 1, the title compound, mp 162—164°C, was obtained.

Example 4

4-(3-(4-Acetyl-3-hydroxy-2-propylphenoxy)propylsulfonyl)-2,3-dichlorobenzeneacetic acid

Step A: *Preparation of 4-(3-(4-acetyl-3-hydroxy-2-propylphenoxy)propylsulfonyl)-2,3-dichlorobenzeneacetic acid, methyl ester*

The product obtained in Step G of Example 1, 1.4 gm, in $CH_2Cl_2$, 35 mL, was treated with m-chloroperbenzoic acid, 1.15 gm, for 2 hours at room temperature. $Ca(OH)_2$, 4 gm, was added and the resulting suspension was stirred for 10 minutes. The solids were filtered off and the filtrate was concentrated *in vacuo* to yield the title compound, mp 115—118°C.

Step B: *Preparation of 4-(3-(4-acetyl-3-hydroxy-2-propylphenoxy)propylsulfonyl)-2,3-dichlorobenzeneacetic acid*

By following Step H of Example 1, but substituting the product of Step A of this example for the ester of Step G of Example 1, the title compound, mp 180—181°C, was obtained.

Example 5

4-(3-(4-Acetyl-3-hydroxy-2-propylphenoxy)-2-hydroxypropylthio)-2,3-dichlorobenzeneacetic acid-S-oxide, methyl ester

Step A: *Preparation of 4-(3-(4-acetyl-3-hydroxy-2-propylphenoxy)-2-hydroxypropylthio)-2,3-dichlorobenzeneacetic acid-S-oxide, methyl ester*

By following Step A of Example 3, but substituting the product of Step A of Example 2 for the ester of Step G of Example 1, the title compound mp 149—152°C, was obtained.

Step B: *Preparation of 4-(3-(4-acetyl-3-hydroxy-2-propylphenoxy)-2-hydroxypropylthio)-2,3-dichlorobenzeneacetic acid-S-oxide*

By following Step H of Example 1 but substituting the product of Step A of this example for the ester of Step G of Example 1, the title compound, mp 166—170°C, was obtained.

Example 6

4-(3-(4-Acetyl-3-hydroxy-2-propylphenoxy)-2-hydroxypropylsulfonyl)-2,3-dichlorobenzeneacetic acid

Step A: *Preparation of 4-(3-(4-acetyl-3-hydroxy-2-propylphenoxy)-2-hydroxypropylsulfonyl)-2,3-dichlorobenzeneacetic acid, methyl ester*

By following Step A of Example 4, but substituting the product of Step A of Example 2 for the ster from Step G of Example 1, the title compound, mp 122—125-C, was obtained.

Step B: *Preparation of 4-(3-(4-acetyl-3-hydroxy-2-propylphenoxy)-2-hydroxypropylsulfonyl)-2,3-dichlorobenzeneacetic acid*

By following Step H of Example 1, but substituting the product from Step A of this example for the ester of Step G of Example 1, the title compound, mp 181—183°C, was obtained.

Example 7

4-(3-(4-Acetyl-3-hydroxy-2-propylphenoxy)propylthio-2-fluorobenzeneacetic acid

Step A: *Preparation of 4-methoxy-2-fluoroacetophenone*

By following Step A of Example 1, but substituting m-fluoroanisole for 2,3-dichloroanisole, the title compound, mp 50—52°C, was obtained.

Step B: *Preparation of 4-methoxy-2-fluorobenzeneacetic acid, methyl ester*

By following Step B of Example 1 but substituting 4-methoxy-2-fluoroacetophenone for 2,3-dichloro-4-methoxyacetophenone, the title compound, bp 112—115°C/l Torr, was obtained.

Step C: *Preparation of 4-hydroxy-2-fluorobenzene acetic acid, methyl ester*

By following Step C of Example 1 but substituting 4-methoxy-2-fluorobenzeneacetic acid, methyl ester for 2,3-dichloro-4-methoxybenzeneacetic acid, methyl ester, 4-hydroxy-2-fluorobenzeneacetic acid was obtained as a sticky solid which was treated with methanolic HCl to yield the title compound as an oil. It was characterized by its NMR spectrum: (ppm) ($CDCl_3$) 3.70 (3H, s, $CH_3O$), 3.55 (2H, s, $CH_2$).

Step D: *Preparation of 2-fluoro-4-dimethylthiocarbamoyloxybenzeneacetic acid, methyl ester*

By following Step D of Example 1 but substituting 4-hydroxy-2-fluorobenzeneacetic acid, methyl ester for 4-hydroxy-2,3-dichlorobenzeneacetic acid, methyl ester, the title compound, mp 113—114°C, was obtained.

Step E: *Preparation of 2-fluoro-4-dimethylcarbamoylthiobenzeneacetic acid, methyl ester*

By following Step E of Example 1 but substituting 2-fluoro-4-dimethylcarbamoyloxybenzeneacetic acid, methyl ester for 2,3-dichloro-4-dimethylthiocarbamoyloxybenzeneacetic acid, methyl ester, the title compound, mp 79—81°C, was obtained.

Step F: *Preparation of the sodium salt of 2-fluoro-4-mercaptobenzeneacetic acid, methyl ester*

By following Step F of Example 1 but substituting 2-fluoro-4-dimethylcarbamoylthiobenzeneacetic acid, methyl ester for 2,3-dichloro-4-dimethylcarbamoylthiobenzeneacetic acid, methyl ester a solution of the title compound was obtained and was used as such in the following Step.

Step G: *Preparation of 4-(3-(4-acetyl-3-hydroxy-2-propylphenoxy)propylthio-2-fluorobenzeneacetic acid, methyl ester*

By following Step G of Example 1 but substituting the product of Step F of this example for the product of Step F of Example 1, the title compound, mp 63—65°C, was obtained.

Step H: *Preparation of 4-(3-(4-acetyl-3-hydroxy-2-propylphenoxy)propylthio-2-fluorobenzeneacetic acid*

By following Step H of Example 1, but substituting the methyl ester of Step G of this example for the methyl ester of Step G of Example 1, the title compound, mp 154—156°C, was obtained.

Example 8

Sodium salt of 4-(3-(4-Acetyl-3-hydroxy-2-propylphenoxy)-2-hydroxypropylthio-2-fluorobenzeneacetic acid, monohydrate

Step A: *Preparation of 4-(3-(4-acetyl-3-hydroxy-2-propylphenoxy)-2-hydroxypropylthio-2-fluorobenzeneacetic acid, methyl ester*

By following Step G of Example 7, but substituting 4-(2,3-epoxypropoxy)-3-propyl-2-hydroxyacetophenone for 4-(3-bromopropoxy)-3-propyl-2-hydroxyacetophenone, the title compound was obtained as an oil.

| | | | | |
|---|---|---|---|---|
| Analysis, calculated: | C, 61.31; | H, 6.04; | F, 4.21; | S, 7.11. |
| Obtained: | C, 61.08; | H, 6.51; | F, 3.93; | S, 6.69. |

Step B: *Preparation of the sodium salt of 4-(3-(4-acetyl-3-hydroxy-2-propylphenoxy)-2-hydroxypropylthio-2-fluorobenzeneacetic acid, monohydrate*

By following Step H of Example 7, but substituting the product of Step A of this example for the ester of Step G of Example 7, the corresponding acid of the title compound was obtained as an oil. It was treated with one equivalent of sodium hydroxide in water to yield, after evaporation of the water, the title compound, mp 75—83°C.

| | | | | |
|---|---|---|---|---|
| Analysis, calculated: | C, 55.45; | H, 5.50; | F, 3.98; | S, 6.73. |
| Obtained: | C, 55.55; | H, 5.56; | F, 4.71; | S, 6.99 |

Example 9

4-(3-(4-Acetyl-3-hydroxy-2-propylphenoxy)-2-hydroxypropylthio)-2-fluorobenzeneacetic acid-S-oxide

Step A: *Preparation of 4-(3-(4-acetyl-3-hydroxy-2-propylphenoxy)-2-hydroxypropylthio)-2-fluorobenzeneacetic acid-S-oxide, methyl ester*

By following Step A of Example 3, but substituting the title compound of Step A of Example 8 for the title compound of Step G of Example 1, the title compound is obtained.

Step B: *Preparation 4-(3-(4-acetyl-3-hydroxy-2-propylphenoxy)-2-hydroxypropylthio)-2-fluorobenzeneacetic acid-S-oxide*

By following Step H of Example 1, but substituting the product of Step A of this example for the ester of Step G of Example 1, the title compound is obtained.

Example 10

4-(3-(4-Acetyl-3-hydroxy-2-propylphenoxy)-2-hydroxypropylsulfonyl)-2-fluorobenzeneacetic acid; and 4-(3-(4-Acetyl-3-hydroxy-2-propylpropyl)-1-propenylsulfonyl)-2-fluorobenzeneacetic acid

Step A: *Preparation of 4-(3-(4-acetyl-3-hydroxy-2-propylphenoxy)-2-hydroxypropylsulfonyl)-2-fluorobenzeneacetic acid, methyl ester*

By following Step A of Example 4, but substituting the product of Step A of Example 8 for the title compound of Step G of Example 1, the title compound, mp 118—119°C, was obtained.

Step B: *Preparation of 4-(3-(4-acetyl-3-hydroxy-2-propylphenoxy)-2-hydroxypropylsulfonyl)-2-fluorobenzeneacetic acid; and 4-(3-(4-acetyl-3-hydroxy-2-propylphenoxy)-1-propenylsulfonyl)-2-fluorobenzeneacetic acid*

By following Step H of Example 1, but substituting the product of Step A of this example for the ester of Step G of Example 1, a mixture was obtained. After recrystallization from diethyl ether, 4-(3-(4-acetyl-3-hydroxy-2-propylphenoxy)-2-hydroxypropylsulfonyl)-2-fluorobenzeneacetic acid, mp 119—120°C, was obtained. The mother liquors were purified by chromatography on silica gel to yield 4-(3-(4-acetyl-3-hydroxy-2-propylphenoxy)-1-propenyl-sulfonyl)-2-fluoro-benzene acetic acid, mp 249—251°C.

## Example 11

4-(3-(4-Acetyl-3-hydroxy-2-propylphenoxy)propylthio-3-fluorobenzeneacetic acid

Step A: *Preparation of 4-dimethylthiocarbamoyloxy-3-fluorobenzeneacetic acid, methyl ester*

By following Step D of Example 7, but substituting 4-hydroxy-3-fluorobenzene acetic acid, methyl ester for 4-hydroxy-2-fluorobenzeneacetic acid, methyl ester, the title compound, was obtained as an oil which was characterized by its NMR spectrum: (ppm) ($CDCl_3$) 3.70 (3H, s, $CH_3O$), 3.60 (2H, s, $CH_2$), 3.40 (3H, s, $CH_3N$), 3.33 (3H, s, $CH_3N$).

Step B: *Preparation of 4-dimethylcarbamoylthio-3-fluorobenzeneacetic acid, methyl ester*

By following Step E of Example 7, but substituting 4-dimethylthiocarbamoyloxy-3-fluorobenzeneacetic acid, methyl ester for 4-dimethylthiocarbamoyloxy-2-fluorobenzeneacetic acid, methyl ester, the title compound, was obtained as an oil which was characterized by its NMR spectrum: (ppm) ($CDCl_3$) 3.70 (3H, s, $CH_3$), 3.63 (2H, s, $CH_2$), 3.07 (6H, s, $(CH_3)_2N$).

Step C: *Preparation of the sodium salt of 3-fluoro-4-mercaptobenzeneacetic acid, methyl ester*

By following Step F of Example 7, but substituting 4-dimethylcarbamoylthio-3-fluorobenzeneacetic acid, methyl ester for fluoro-4-dimethylcarbamoylthiobenzeneacetic acid, methyl ester, a solution of the title compound was obtained and was used as such in the following Step.

Step D: *Preparation of 4-(3-(4-acetyl-3-hydroxy-2-propylphenoxy)-propylthio)-3-fluorobenzeneacetic acid, methyl ester*

By following Step G of Example 7, but substituting the product of Step C of this example for the product of Step F of Example 1, the title compound was obtained as an oil which was characterized by its NMR spectrum: (ppm) ($CDCl_3$) 12.70 (1H, s, phenolic OH), 7.53 (1H, d, J=9Hz, H ortho to acetyl), 6.33 (1H, d, J=9Hz H meta to acetyl), 4.13 (2H, t, J=7Hz, $CH_2O$), 3.67 (3H, s, $CH_3O$), 3.57 (2H, s, $CH_2$), 2.53 (3H, s, $CH_3CO$).

Step E: *Preparation of 4-(3-(4-acetyl-3-hydroxy-2-propylphenoxy)-propylthio)-3-fluorobenzeneacetic acid*

By following Step H of Example 7, but substituting the methyl ester of Step D of this example for the product of Step G of Example 7, the title compound, mp 84—85°C, was obtained.

## Example 12

4-(3-(4-Acetyl-3-hydroxy-2-propylphenoxy)-2-hydroxypropylthio)-3-fluorobenzeneacetic acid

Step A: *Preparation of 4-(3-(4-acetyl-3-hydroxy-2-propylphenoxy)-2-hydroxypropylthio)-3-fluorobenzeneacetic acid, methyl ester*

By following Step D of Example 11, but substituting 4-(2,3-epoxypropoxy)-3-propyl-2-hydroxyacetophenone for 4-(3-bromopropoxy)-3-propyl-2-hydroxyacetophenone, the title compound was obtained as an oil which was characterized by its NMR spectrum: (ppm) ($CDCl_3$) 12.70 (1H, s), 7.53 (1H, d, J=9Hz), 6.33 (1H, d, J=9Hz), 3.67 (3H, s), 3.57 (2H, s), 2.53 (3H, s).

Step B: *Preparation of 4-(3-(4-acetyl-3-hydroxy-2-propylphenoxy)-2-hydroxypropylthio-3-fluorobenzeneacetic acid*

By following Step H of Example 1, but substituting the title compound of Step A of this example for 4-(3-(4-acetyl-3-hydroxy-2-propylphenoxy)-propylthio-2,3-dichlorobenzeneacetic acid, methyl ester, the title compound, mp 113—114°C, was obtained.

## Example 13

4-(3-(4-Acetyl-3-hydroxy-2-propylphenoxy)-propylthio-3-chlorobenzeneacetic acid

Step A: *Preparation of 4-dimethylthiocarbamoyloxy-3-chlorobenzeneacetic acid, methyl ester*

By following Step D of Example 7, but substituting 4-hydroxy-3-chlorobenzeneacetic acid, methyl ester for 4-hydroxy-2-fluorobenzene acetic acid, methyl ester, the title compound, mp 76—78°C was obtained.

Step B: *Preparation of 4-dimethylcarbamoylthio-3-chlorobenzeneacetic acid, methyl ester*

By following Step E of Example 7, but substituting 4-dimethylthiocarbamoyloxy-3-chlorobenzeneacetic acid, methyl ester for 4-dimethylthiocarbamoyloxy-2-fluorobenzeneacetic acid, methyl ester, the title

compound, was obtained as an oil which was characterized by its NMR spectrum; (ppm) ($CDCl_3$) 3.60 (3H, s), 3.50 (2H, s), 2.90 (6H, s).

Step C: *Preparation of the sodium salt of 3-chloro-4-mercaptobenzeneacetic acid, methyl ester*

By following Step F of Example 7, but substituting 4-dimethylcarbamoylthio-3-chlorobenzeneacetic acid, methyl ester for 4-dimethylcarbamoylthio-2-fluorobenzeneacetic acid, methyl ester, a solution of the title compound was obtained and was used as such in the following Step.

Step D: *Preparation of 4-(3-(4-acetyl-3-hydroxy-2-propylphenoxy)-propylthio)-3-chlorobenzeneacetic acid, methyl ester*

By following Step G of Example 7, but substituting the product of Step C of this example for the product of Step F of Example 1, the title compound, mp 66—68°C, was obtained.

Step E: *Preparation of 4-(3-(4-acetyl-3-hydroxy-2-propylphenoxy)-propylthio)-3-chlorobenzene acetic acid*

By following Step H of Example 7, but substituting the methyl ester of Step D of this example for the product of Step G of Example 7, the title compound, mp 79—81°C, was obtained.

Example 14

4-(3-(4-Acetyl-3-hydroxy-2-propylphenoxy)-propylthio)-3-chlorobenzeneacetic acid-S-oxide

Step A: *Preparation of 4-(3-(4-acetyl-3-hydroxy-2-propylphenoxy)-propylthio)-3-chlorobenzene acetic acid-S-oxide, methyl ester*

By following Step A of Example 3, but substituting the title compound of Step D of Example 13 for the title compound of Step G of Example 1, the title compound, mp 90—91°C, was obtained.

Step B: *Preparation of 4-(3-(4-acetyl-3-hydroxy-2-propylphenoxy)-propylthio)-3-chlorobenzene acetic acid-S-oxide*

By following Step H of Example 1, but substituting the product of Step A of this example for the ester of Step G of Example 1, the title compound, mp 130—132°C, was obtained.

Example 15

4-(3-(4-Acetyl-3-hydroxy-2-propylphenoxy)-propylsulfonyl-3-chlorobenzeneacetic acid

Step A: *Preparation of 4-(3-(4-acetyl-3-hydroxy-2-propylphenoxy)-propylsulfonyl)-3-chlorobenzeneacetic acid, methyl ester*

By following Step A of Example 4, but substituting the product of Step D of Example 13 for the title compound of Step G of Example 1, the title compound, mp 98—100°C, was obtained

Step B: *Preparation of 4-(3-(4-acetyl-3-hydroxy-2-propylphenoxy)-propylsulfonyl)-3-chlorobenzeneacetic acid*

By following Step H of Example 1, but substituting the product of Step A of this example for the ester of Step G of Example 1, the title compound, mp 132—134°C, was obtained.

Example 16

4-(3-(4-Acetyl-3-hydroxy-2-propylphenoxy)-2-hydroxypropylthio)-3-chlorobenzeneacetic acid

Step A: *Preparation of 4-(3-(4-acetyl-3-hydroxy-2-propylphenoxy)-2-hydroxypropylthio)-3-chlorobenzene-acetic acid, methyl ester*

By following Step D of Example 13, but substituting 4-(2,3-epoxypropoxy)-3-propyl-2-hydroxy-acetophenone for 4-(3-bromopropoxy)-3-propyl-2-hydroxyacetophenone, the title compound, mp 55—58°C, was obtained.

Step B: *Preparation of 4-(3-(4-acetyl-3-hydroxy-2-propylphenoxy)-2-hydroxypropylthio)-3-chlorobenzene-acetic acid*

By following Step H of Example 1, but substituting the title compound of Step A of this example for 4-(3-(4-acetyl-3-hydroxy-propylphenoxy)-propylthio-2,3-dichlorobenzeneacetic acid, methyl ester, the title compound, mp 128—129°C, was obtained.

Example 17

4-(3-(4-Acetyl-3-hydroxy-2-propylphenoxy)-2-hydroxypropylthio)-3-chlorobenzeneacetic acid-S-oxide

Step A: *Preparation of 4-(3-(4-acetyl-3-hydroxy-2-propylphenoxy)-2-hydroxypropylthio)-3-chloro-benzeneacetic acid-S-oxide, methyl ester*

By following Step A of Example 3, but substituting the title compound of Step A of Example 16 for the title compound of Step G of Example 1, the title compound, mp 103—104°C, was obtained.

Step B: *Preparation of 4-(3-(4-acetyl-3-hydroxy-2-propylphenoxy)-2-hydroxypropylthio)-3-chlorobenzene-acetic acid-S-oxide*

By following Step H of Example 1, but substituting the product of Step A of this example for the ester of Step G of Example 1, the title compound, mp 119—123°C, was obtained.

## Example 18

4-(3-(4-Acetyl-3-hydroxy-2-propylphenoxy)-2-hydroxypropylsulfonyl)-3-chlorobenzeneacetic acid

Step A: *Preparation of 4-(3-(4-acetyl-3-hydroxy-2-propylphenoxy)-2-hydroxypropylsulfonyl)-3-chlorobenzeneacetic acid, methyl ester*

By following Step A of Example 4, but substituting the title compound of Step A of Example 16 for the title compound of Step G of Example 1, the title compound, mp 96—98°C, was obtained.

Step B: *Preparation of 4-(3-(4-acetyl-3-hydroxy-2-propylphenoxy)-2-hydroxypropylsulfonyl)-3-chlorobenzeneacetic acid*

By following Step H of Example 1, but substituting the product of Step A of this example for the ester of Step G of Example 1, the title compound, mp 110—112°C, was obtained.

## Example 19

4-(3-(4-Acetyl-3-hydroxy-2-propylphenoxy)-propylthio)-benzeneacetic acid

Step A: *Preparation of 4-(3-(4-acetyl-3-hydroxy-2-propylphenoxy)-propylthio)benzeneacetic acid methyl ester*

By following Step G of Example 1, but substituting 4-mercaptobenzeneacetic acid, methyl ester for 2,3-dichloro-4-mercaptobenzeneacetic acid, methyl ester, the title compound, was obtained as an oil which was characterized by its NMR spectrum: (ppm) ($CDCl_3$) 12.67 (1H, s), 7.63 (1H, d, J=9Hz), 6.40 (1H, d, J=9Hz), 4.13 (2H, t, J=6Hz), 3.70 (3H, s), 3.60 (2H, s), 2.57 (3H, s).

Step B: *Preparation of 4-(3-(4-acetyl-3-hydroxy-2-propylphenoxy)-propylthio)benzeneacetic acid*

By following Step H of Example 1, but substituting the product of Step A of this example for the title compound of Step G of Example 1, the title compound, mp 94°C, was obtained.

## Example 20

4-(3-(4-Acetyl-3-hydroxy-2-propylphenoxy)-propylsulfonyl)-benzeneacetic acid

Step A: *Preparation of 4-(3-(4-acetyl-3-hydroxy-2-propylphenoxy)-propylsulfonyl)-benzeneacetic acid, methyl ester*

By following Step A of Example 4, but substituting the title compound of Step A of Example 19 for the title compound of Step G of Example 1, the title compound, was obtained as an oil which was characterized by its NMR spectrum: (ppm) ($CDCl_3$) 12.67 (1H, s), 7.63 (1H, d, J=9Hz), 6.40 (1H, d, J=9Hz), 4.13 (2H, t, J=6Hz), 3.70 (3H, s), 3.60 (2H, s), 2.57 (3H, s).

Step B: *Preparation of 4-(3-(4-acetyl-3-hydroxy-2-propylphenoxy)-propylsulfonyl)-benzeneacetic acid*

By following Step H of Example 1, but substituting the title compound of Step A of this example for the title compound of Step G of Example 1, the title compound, mp 132—134°C, was obtained.

## Example 21

4-(3-(4-Acetyl-3-hydroxy-2-propylphenoxy)-2-methyl-propylthio)-benzeneacetic acid

Step A: *Preparation of 4-(3-bromo-2-methylpropoxy)-2-hydroxy-3-propylacetophenone*

To a solution of 2,4-dihydroxy-3-propylacetophenone, 10 gm, in methylethyl ketone, 250 mL, was added successively 2-methyl-1,3-dibromopropane, 33.4 gm, and potassium carbonate, 11 gm. The suspension was stirred at reflux for 4 hours. The solids were filtered off, and were washed thoroughly with acetone. The filtrate was evaporated to dryness to yield the title compound as an oil after purification by chromatography on silica gel. NMR (ppm) ($CDCl_3$) 3.95 (2H, d, J=7Hz), 3.55 (2H, d, J=7Hz), 1.20 (3H, d, J=7Hz).

Step B: Preparation of 4-(3-(4-acetyl-3-hydroxy-2-propylphenoxy)-2-methylpropylthio)-benzeneacetic acid, methyl ester

By following Step A of Example 19, but substituting the title compound of Step A of this example for 4-(3-(bromopropoxy)-3-propyl-2-hydroxyacetophenone, the title compound was obtained as an oil, NMR (ppm) ($CDCl_3$) 12.65 (1H, s), 7.58 (1H, d, J=9Hz), 6.40 (1H, d, J=9Hz), 3.60 (3H, s), 3.50 (2H, s) 2.55 (3H, s).

Step C: *Preparation of 4-(3-(4-acetyl-3-hydroxy-2-propylphenoxy)-2-methylpropylthio)-benzeneacetic acid*

By following Step H of Example 1, but substituting the title compound of Step B of this example for the title compound of Step G of Example 1, the title compound, mp 96—98°C, was obtained.

Example 22

4-(3-(4-Acetyl-3-hydroxy-2-propylphenoxy)-2-hydroxypropylthio)-benzeneacetic acid

Step A: *Preparation of 4-(3-(4-acetyl-3-hydroxy-2-propylphenoxy)-2-hydroxypropylthio)-benzeneacetic acid methyl ester*

By following Step A of Example 2, but substituting 4-mercaptobenzeneacetic acid, methyl ester for the title compound of Step F of Example 1, the title compound, was obtained as an oil. NMR (ppm) ($CDCl_3$) 12.77 (1H, s), 7.67 (1H, d, J=9Hz), 7.27 (2H, d, J=9Hz), 6.93 (2H, d, J=9Hz), 6.50 (1H, d, J=9Hz), 3.67 (3H, s).

Step B: *Preparation of 4-(3-(4-acetyl-3-hydroxy-2-propylphenoxy)-2-hydroxypropylthio)-benzeneacetic acid*

By following Step H of Example 1, but substituting the title compound of Step A of this example for the title compound of Step G of Example 1, the title compound, mp 105.4°C, was obtained.

Example 23

4-(3-(4-Acetyl-3-hydroxy-2-propylphenoxy)-2-hydroxypropylthio)-3-fluorobenzoic acid

Step A: *Preparation of 4-dimethylthiocarbamoyloxy-3-fluorobenzoic acid, methyl ester*

By following Step D of Example 7, but substituting 4-hydroxy-3-fluoro-benzoic acid, methyl ester for 4-hydroxy-2-fluorobenzeneacetic acid, methyl ester, the title compound, mp 103—104°C, was obtained.

Step B: *Preparation of 4-dimethylcarbamoylthio-3-fluorobenzoic acid, methyl ester*

By following Step E of Example 7, but substituting 4-dimethylthiocarbamoyloxy-3-fluorobenzoic acid, methyl ester for 4-dimethylthiocarbamoyloxy-2-fluorobenzeneacetic acid, methyl ester, the title compound, mp 72—74°C, was obtained.

Step C: *Preparation of the sodium salt 4-mercapto-3-fluorobenzoic acid, methyl ester*

By following Step F of Example 7, but substituting 4-dimethylcarbamoylthio-3-fluorobenzoic acid, methyl ester doe 4-dimethylcarbamoylthio-2-fluorobenzeneacetic acid, methyl ester, a solution of the title compound was obtained and was used as such in the following step.

Step D: *Preparation of 4-(3-(4-acetyl-3-hydroxy-2-propylphenoxy)-2-hydroxypropylthio)-3-fluorobenzoic acid, methyl ester*

By following Step A of Example 12, but substituting 4-mercapto-3-fluorobenzoic acid, methyl ester for 4-mercapto-3-fluorobenzeneacetic acid, methyl ester, the title compound, mp 123—125°C, was obtained.

Step E: *Preparation of 4-(3-(4-acetyl-3-hydroxy-2-propylphenoxy)-2-hydroxypropylthio)-3-fluorobenzoic acid*

By following Step H of Example 1, but substituting the title compound of Step D of this example for the title compound of Step G of Example 1, the title compound, mp 171—175°C, was obtained.

Example 24

4-(3-(4-Acetyl-3-hydroxy-2-propylphenoxy)-2-hydroxypropylthio)-3-fluorobenzoic acid-S-oxide

Step A: *Preparation of 4-(3-(4-acetyl-3-hydroxy-2-propylphenoxy)-2-hydroxypropylthio)-3-fluorobenzoic acid-S-oxide, methyl ester*

By following Step A of Example 3 but substituting the title compound of Step D of Example 23 for the title compound of Step G of Example 1, the title compound, mp 103—104°C, was obtained.

Step B: *Preparation of 4-(3-(4-acetyl-3-hydroxy-2-propylphenoxy)-2-hydroxypropylthio)-3-fluorobenzoic acid-S-oxide*

By following Step H of Example 1, but substituting the title compound of Step A of this example for the title compound of Step G of Example 1, the title compound, mp 72—74°C, was obtained.

Example 25

4-(3-(4-Acetyl-3-hydroxy-2-propylphenoxy)-2-hydroxy-propylsulfonyl)-3-fluorobenzoic acid

Step A: *Preparation of 4-(3-(4-acetyl-3-hydroxy-2-propylphenoxy)-2-hydroxypropylsulfonyl)-3-fluorobenzoic acid, methyl ester*

By following the indications of Step A of Example 4, but substituting the title compound of Step D of Example 23 for the title compound of Step G of Example 1, the title compound was obtained as an oil. NMR (ppm) ($CDCl_3$) 12.75 (1H, s), 7.85 (1H, d, J=9Hz), 6.35 (1H, d, J=9Hz), 3.95 (3H, s), 2.54 (3H, s).

Step B: *Preparation of 4-(3-(4-acetyl-3-hydroxy-2-propylphenoxy)-2-hydroxypropylsulfonyl)-3-fluorobenzoic acid*

By following Step H of Example 1, but substituting the title compound of Step A of this example for the

title compound of Step G of Example 1, the title compound, mp 128—129°C, was obtained.

Example 26

4-(3-(4-Acetyl-3-hydroxy-2-propylphenoxy)-propylthio-3-fluorobenzoic acid

Step A: *Preparation of 4-(3-(4-Acetyl-3-hydroxy-2-propylphenoxy)-propylthio-3-fluorobenzoic acid, methyl ester*

By following Step D of Example 23, but substituting 4-(3-bromopropoxy)-3-propyl-2-hydroxyacetophenone for 4-(2,3-epoxypropoxy)-3-propyl-2-hydroxyacetophenone, the title compound, mp 62—65°C, was obtained.

Step B: *Preparation of 4-(3-(4-acetyl-3-hydroxy-2-propylphenoxy)-propylthio-3-fluorobenzoic acid*

By following Step H of Example 1, but substituting the title compound of Step A of this example for the title compound of Step G of Example 1, the title compound, mp 128—129°C, was obtained.

Example 27

4-(3-(4-Acetyl-3-hydroxy-2-propylphenoxy)-propylthio)-3-fluorobenzoic acid-S-oxide

Step A: *Preparation of 4-(3-(4-acetyl-3-hydroxy-2-propylphenoxy)-propylthio)-3-fluorobenzoic acid-S-oxide, methyl ester*

By following Step A of Example 3, but substituting the title compound of Step A of Example 26 for the title compound of Step G of Example 1, the title compound, mp 108—109°C, was obtained.

Step B: *Preparation of 4-(3-(4-acetyl-3-hydroxy-2-propylphenoxy)-propylthio)-3-fluorobenzoic acid-S-oxide*

By following Step H of Example 1, but substituting the title compound of Step A of this example for the title compound of Step G of Example 1, the title compound, mp 175—176°C, was obtained.

Example 28

4-(3-(4-Acetyl-3-hydroxy-2-propylphenoxy)-propylsulfonyl)-3-fluorobenzoic acid

Step A: *Preparation of 4-(3-(4-acetyl-3-hydroxy-2-propylphenoxy)-propylsulfonyl)-3-fluorobenzoic acid, methyl ester*

By following Step A of Example 4, but substituting the title compound of Step A of Example 26 for the title compound of Step G of Example 1, the title compound, mp 102—103°C, was obtained.

Step B: *Preparation of 4-(3-(4-acetyl-3-hydroxy-2-propylphenoxy)-propylsulfonyl)-3-fluorobenzoic acid*

By following Step H of Example 1, but substituting the title compound of Step A of this example for the title compound of Step G of Example 1, the title compound, mp 181—182°C, was obtained.

Example 29

4-(3-(4-Acetyl-3-hydroxy-2-propylphenoxy)-propylcyanamido)-benzeneacetic acid

Step A: *Preparation of 4-cyanamidobenzeneacetic acid, methyl ester*

To a mixture of p-aminobenzeneacetic acid, methyl ester, 1.65 gm, and sodium acetate, 1.4 gm, in $H_2O$, 15 mL, cooled at 0°C, was added CNBr, 1.3 gm. The suspension was allowed to warm to room temperature and was stirred overnight. The title compound was then filtered off and has mp 77—79°C after crystallization from EtOAc/hexane.

Step B: *Preparation of 4-(3-(4-acetyl-3-hydroxy-2-propylphenoxy)-propylcyanamido)-benzeneacetic acid, methyl ester*

By following Step G of Example 1, but substituting 4-cyanamidobenzeneacetic acid, methyl ester for 4-mercapto-2,3-dichlorobenzeneacetic acid, methyl ester, the title compound, mp 92—93°C, was obtained.

Step C: *Preparation of 4-(3-(4-acetyl-3-hydroxy-2-propylphenoxy)-propylcyanamido)-benzeneacetic acid*

By following Step H of Example 1, but substituting the title compound of Step B of this example for the title compound of Step G of Example 1, the title compound, mp 130—131°C, was obtained.

Example 30

4-(3-(4-Acetyl-3-hydroxy-2-propylphenoxy)-propylthio) delta-hydroxybenzenebutanol

Step A: *Preparation of I: 4-(3-bromopropylthio)-delta-oxo-benzenebutanol and II: 4-(3-bromopropylthio)-delta-hydroxybenzenebutanol*

4-(3-Bromopropylthio)-gamma-oxobenzenebutanoic acid (3.4 g, 10.3 mmoles) was dissolved in THF (25 ml) and cooled to −15°. Borane-THF complex (10 ml, 10.4 mmoles) was added dropwise over one hour. The solution warmed to room temperature with stirring over twelve hours. The reaction mixture was diluted with methanol and stirred for thirty minutes. The solution was evaporated to dryness, dissolved in

toluene, cooled to 0°C and treated with an excess of diazomethane. After twelve hours the reaction was evaporated to dryness and purified by HPLC to yield the title compounds:

I mass spectrum $M^+$ 298
II mass spectrum $M^+$ 300

Step B: *Preparation of 4-(3-(4-Acetyl-3-hydroxy-2-propylphenoxy)-propylthio)delta-hydroxybenzenebutanol*

The compound II from Step A above, (2.0 g, 6.25 mmoles), 2,4-dihydroxy-3-propylacetophenone, (1.94 g, 10 mmoles) and potassium carbonate (2.76 g, 20 mmoles) were heated at reflux in methyl ethyl ketone (20 ml) for six hours. The reaction mixture was cooled to room temperature, filtered and evaporated to dryness. HPLC afforded the title compound.

Analysis: calculated: C, 66.64; H, 7.46; S, 7.41.
Obtained: C, 66.65; H, 7.40; S, 7.30.

Example 31

4-(3-(4-Acetyl-3-hydroxy-2-propylphenoxy)-propylsulfonyl)-delta-hydroxybenzenebutanol

The compound of Example 30, Step B, (1.0 g, 2.32 mmole) was dissolved in chloroform and cooled to 0°. A solution of m-chloroperbenzoic acid (1.0 g, 4.8 meq.) in chloroform was added dropwise. The reaction stirred at 0°C for one hour. Calcium hydroxide (590 mg) was added and the reaction stirred twenty-five minutes. The mixture was filtered and evaporated to dryness. Crystallization of the residue from ethyl acetate-hexane afforded the title compound, m.p. 74—75°.

Analysis, calculated: C, 62.05; H, 6.94; S, 6.90.
Obtained: C, 62.07; H, 6.92; S, 6.72.

Similarly there may be prepared the following compounds.

Example 32

D,L-4-(3-(4-acetyl-3-hydroxy-2-propylphenoxy)propylthio)-alpha-hydroxybenzeneacetic acid

Analysis, calculated: C, 63.29; H, 6.04; S, 7.68.
Observed: C, 63.26; H, 6.13; S, 7.50.

Example 33

D,L-4-(3-(4-acetyl-3-hydroxy-2-propylphenoxy)propylsulfonyl)-alpha-hydroxybenzeneacetic acid

Analysis, calculated: C, 58.66;, H, 5.82; S, 7.12.
Observed: C, 58.75; H, 5.85; S, 6.97.

Example 34

D,L-4-(3-(4-acetyl-3-hydroxy-2-propylphenoxy)propyloxy)-alpha-hydroxybenzeneacetic acid

Analysis, calculated: C, 65.66; H, 6.51.
Observed: C, 65.79; H, 6.56.

Example 35

D,L-4-(3-(4-acetyl-3-hydroxy-2-propylphenoxy)propylthio)-3-bromo-alpha-hydroxybenzeneacetic acid

Example 36

D,L-4-(3-(4-acetyl-3-hydroxy-2-propylphenoxy)propylthio)-3-fluoro-alpha-hydroxybenzeneacetic acid

Example 37

D,L-2-(4-(3-(4-acetyl-3-hydroxy-2-propylphenoxy)propyloxy)-alpha-hydroxybenzeneacetic acid

Example 38

Methyl 4-(4-(3-(4-acetyl-3-hydroxy-2-propylphenoxy)-propylthio)phenyl)-3-methyl-3(E)-butenoate

Example 39

4-(4-(3-(4-acetyl-3-hydroxy-2-propylphenoxy)propylthio)phenyl-3-methyl-3(E)-butenoic acid

Example 40

Methyl 4-(4-(3-(4-acetyl-3-hydroxy-2-propylphenoxy)-propylthio)phenyl)-3-methyl-3(Z)-butenoate

Example 41

4-(3-(4-acetyl-3-hydroxy-2-propylphenoxy)propylthio)phenyl)-3-methyl-3(Z)-butenoic acid

It is understood that for those compounds which contain asymmetric centers, the present invention includes the racemic mixture as well as the individual resolved optical isomers.

**Claims for the contracting states: BE CH DE FR GB IT LI LU NL SE**

1. a compound having either of the following formulae

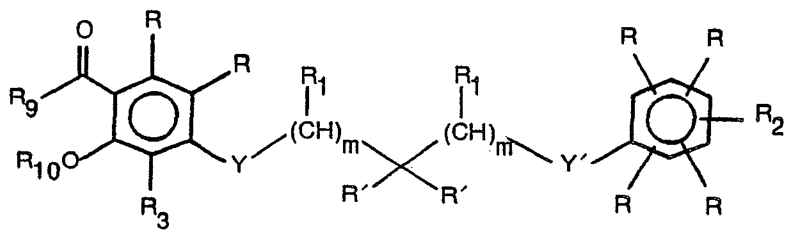

XI

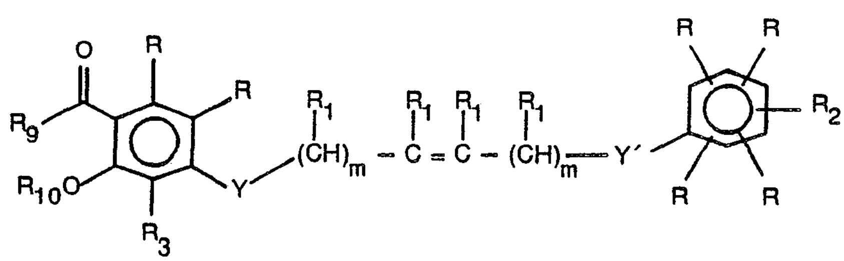

XII

in which

each R, independently of the others, is H, OH, $C_{1-6}$ alkyl; $C_{2-6}$ alkyl; $C_{2-6}$ alkenyl; trifluoromethyl; $C_{1-6}$ alkoxy; $C_{1-6}$ thioalkyl; phenyl; phenyl substituted by alkyl of 1 to 3 carbon atoms or by halogen; benzyl; phen($C_{2-4}$ alkyl); halogen, $COOR_4$ where $R_4$ is H or $C_{1-6}$ alkyl; CN; trifluoromethylthio; or nitro;

each R′ is independently $R_4$; $OR_4$; $COOR_4$; $N(R_4)_2$; $SR_4$; $CH_2OR_4$; CHO; or together R′ and R′ are O; $CH_2$; or

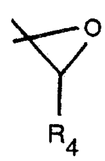

;

each of Y and Y′, which are the same as or different from one another, is oxygen, sulfur, sulfoxide, sulfone;

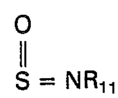

where $R_{11}$ is H, $C_{1-4}$ alkyl, $C_{1-4}$ alkanoyl, phenylsulfonyl or tosyl;

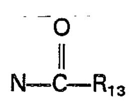

where $R_{13}$ is $C_{1-4}$ alkyl or $C_{1-4}$ alkoxy; N—CN; $CH_2$ or C = O, but with the proviso that Y′ is not oxygen unless Y is oxygen, sulfur, sulfoxide, sulfone or cyanamido and $R_2$ is

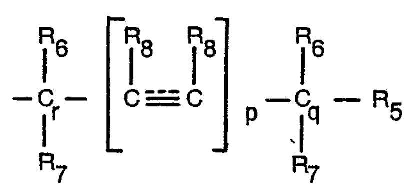

where at least one $R_7$ is OH;

each $R_1$, independently of the others, is hydrogen or $C_{1-3}$ alkyl;

each m, independently of the others, is 0, 1, 2 or 3;

$R_2$ is

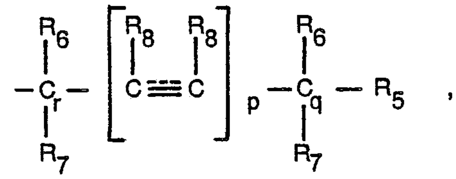

where

each $R_6$, independently of the others, is H or $C_{1-4}$ alkyl;

each $R_7$, independently of the others, is H, OH, or $C_{1-4}$ alkyl;

each $_8$, independently of the others, is H or $C_{1-4}$ alkyl; and is absent when a triple bond is present;

$R_5$ is $COOR_4$, $CH_2OH$; CHO; tetrazole; hydroxymethylketone; $CONHSO_2R_{14}$; CN; $CON(R_7)_2$; or $COOR_{15}$ where $R_{15}$ is:

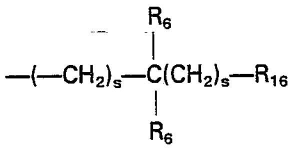

where each s is independently 0—3 and $R_{16}$ is (A) a monocyclic or bicyclic heterocyclic radical containing from 3 to 12 nuclear carbon atoms and 1 or 2 nuclear nitrogen atoms or a nuclear N and a nuclear 5 atom; each ring in the heterocyclic radical being formed of 5 or 6 atoms, or (B) a radical W—$R_{17}$ where W is O, S, or NH and $R_{17}$ contains up to 21 carbon atoms and is a hydrocarbon radical or an acyl radical of an organic acyclic or monocyclic carboxylic acid containing not more than 1 heteroatom in the ring;

each of r and q, independently of the other, is 0 or an integer from 1 to 6, provided that r+q does not exceed 10;

p is 0 or 1;

$R_3$ is $C_{1-6}$ alkyl or $C_{3-6}$ alkenyl;

$R_9$ is OH, $C_{1-6}$ alkyl or $C_{1-6}$ alkoxy;

$R_{10}$ is H; $R_4CO$— or $R_4OCH_2$—;

$R_{14}$ is $C_{1-6}$ alkyl, phenyl, phenyl substituted by $C_{1-3}$ alkyl or alkoxy groups, halogen, hydroxy, haloalkyl, COOH, CN, formyl, $C_{1-6}$ acyl or $C_{1-4}$ perfluoroalkyl; and all carbon chains in all radicals are straight or branched; or a compound that is a pharmaceutically acceptable salt or acid-addition salt thereof.

2. A compound as claimed in Claim 1 in which Y is oxygen and Y' is suylfur, sulfoxide, sulfone, amino or cyanamido.

3. A compound as claimed in Claim 1 in which Y' is oxygen and Y is oxygen, sulfur, sulfoxide, sulfone or cyanamido and $R_2$ is

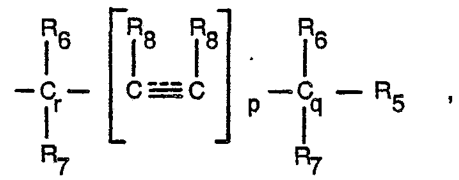

where at least one $R_7$ is OH.

4. The following compounds claimd in Claim 1:—

4-(3-(4-Acetyl-3-hydroxy-2-propylphenoxy)propylthio)-2,3-dichlorobenzeneacetic acid
4-(3-(4-Acetyl-3-hydroxy-2-propylphenoxy)-2-hydroxypropylthio)-2,3-dichlorobenzeneacetic acid
4-(3-(4-Acetyl-3-hydroxy-2-propylphenoxy)propylthio-2,3-dichlorobenzeneacetic acid-S-oxide
4-(3-(4-Acetyl-3-hydroxy-2-propylphenoxy)propylsulfonyl)-2,3-dichlorobenzeneacetic acid
4-(3-(4-Acetyl-3-hydroxy-2-propylphenoxy)-2-hydroxypropylthio)-2,3-dichlorobenzeneacetic acid-S-oxide
4-(3-(4-Acetyl-3-hydroxy-2-propylphenoxy)-2-hydroxypropylsulfonyl)-2,3-dichlorobenzeneacetic acid
4-(3-(4-Acetyl-3-hydroxy-2-propylphenoxy)propylthio)-2-fluorobenzeneacetic acid
4-(3-(4-Acetyl-3-hydroxy-2-propylphenoxy)-2-hydroxypropylthio)-2-fluorobenzeneacetic acid
4-(3-(4-Acetyl-3-hydroxy-2-propylphenoxy)-2-hydroxypropylthio)-2-fluorobenzeneacetic acid-S-oxide
4-(3-(4-Acetyl-3-hydroxy-2-propylphenoxy)-2-hydroxypropylsulfonyl)-2-fluorobenzeneacetic acid
4-(3-(4-Acetyl-3-hydroxy-2-propylphenoxy)-1-propenylsulfonyl)-2-fluorobenzeneacetic acid
4-(3-(4-Acetyl-3-hydroxy-2-propylphenoxy)propylthio)-3-fluorobenzeneacetic acid
4-(3-(4-Acetyl-3-hydroxy-2-propylphenoxy)-2-hydroxypropylthio)-3-fluorobenzeneacetic acid
4-(3-(4-Acetyl-3-hydroxy-2-propylphenoxy)-propylthio)-3-chlorobenzeneacetic acid
4-(3-(4-Acetyl-3-hydroxy-2-propylphenoxy)-propylthio)-3-chlorobenzeneacetic acid-S-oxide
4-(3-(4-Acetyl-3-hydroxy-2-propylphenoxy)-propylsulfonyl)-3-chlorobenzeneacetic acid
4-(3-(4-Acetyl-3-hydroxy-2-propylphenoxy)-2-hydroxypropylthio)-3-chlorobenzeneacetic acid
4-(3-(4-Acetyl-3-hydroxy-2-propylphenoxy)-2-hydroxypropylthio)-3-chlorobenzeneacetic acid-S-oxide

4-(3-(4-Acetyl-3-hydroxy-2-propylphenoxy)-2-hydroxypropylsulfonyl)-3-chlorobenzeneacetic acid
4-(3-(4-Acetyl-3-hydroxy-2-propylphenoxy)-propylthio)benzeneacetic acid
4-(3-(4-Acetyl-3-hydroxy-2-propylphenoxy)-propylsulfonyl)-benzeneacetic acid
4-(3-(4-Acetyl-3-hydroxy-2-propylphenoxy)-2-methylpropylthio)-benzeneacetic acid
4-(3-(4-Acetyl-3-hydroxy-2-propylphenoxy)-2-hydroxypropylthio)-benzeneacetic acid
4-(3-(4-Acetyl-3-hydroxy-2-propylphenoxy)-2-hydroxypropylthio)-3-fluorobenzoic acid
4-(3-(4-Acetyl-3-hydroxy-2-propylphenoxy)-2-hydroxypropylthio)-3-fluorobenzoic acid-S-oxide
4-(3-(4-Acetyl-3-hydroxy-2-propylphenoxy)-2-hydroxypropylsulfonyl)-3-fluorobenzoic acid
4-(3-(4-Acetyl-3-hydroxy-2-propylphenoxy)-propylthio)-3-fluorobenzoic acid-S-oxide, methyl ester
4-(3-(4-Acetyl-3-hydroxy-2-propylphenoxy)-propylsulfonyl)-3-fluorobenzoic acid
4-(3-(4-Acetyl-3-hydroxy-2-propylphenoxy)-propylcyanamido)-benzeneacetic acid
4-(3-(4-Acetyl-3-hydroxy-2-propylphenoxy)-propylthio)delta-hydroxybenzenebutanol
4-(3-(4-Acetyl-3-hydroxy-2-propylphenoxy)-propylsulfonyl)-delta-hydroxybenzenebutanol
4-(3-(4-acetyl-3-hydroxy-2-propylphenoxy)-propylthio)-alpha-hydroxybenzeneacetic acid
4-(3-(4-acetyl-3-hydroxy-2-propylphenoxy)-propylsulfonyl)-alpha-hydroxybenzeneacetic acid
4-(3-(4-acetyl-3-hydroxy-2-propylphenoxy)-propyloxy)-alpha-hydroxybenzeneacetic acid
4-(3-(4-acetyl-3-hydroxy-2-propylphenoxy)-propylthio)-3-bromo-alpha-hydroxybenzeneacetic acid
4-(3-(4-acetyl-3-hydroxy-2-propylphenoxy)-propylthio)-3-fluoro-alpha-hydroxybenzeneacetic acid
2-(3-(4-acetyl-3-hydroxy-2-propylphenoxypropyloxy)-alpha-hydroxybenzeneacetic acid
Methyl 4-(4-(3-(4-acetyl-3-hydroxy-2-propylphenoxy)propylthio)phenyl)-3-methyl-3(E)-butenoate
4-(4-(3-(4-acetyl-3-hydroxy-2-propylphenoxy)propylthio)phenyl)-3-methyl-3(E)-butenoic acid
Methyl 4-(4-(3-(4-acetyl-3-hydroxy-2-propylphenoxy)propylthio)phenyl)-3-methyl-3(Z)-butenoate
4-(4-(3-(4-acetyl-3-hydroxy-2-propylphenoxy)propylthio)phenyl)-3-methyl-3(Z)-benzoic acid.

5. 4-(3-(4-Acetyl-3-hydroxy-2-propylphenoxy)propylthio)-3-fluorobenzeneacetic acid.

6. 4-(3-(4-Acetyl-3-hydroxy-2-propylphenoxy)-propylthio)benzeneacetic acid.

7. 4-(3-(4-Acetyl-3-hydroxy-2-propylphenoxy)-propylthio)delta-hydroxybenezenebutanol.

8. 4-(3-(4-Acetyl-3-hydroxy-2-propyl-phenoxy)-propylsulfonyl)-delta-hydroxybenzenebutanol.

9. A pharmaceutical composition useful for antagonizing leukotrienes in mammals, especially humans, containing an effective amount of a compound as claimed in any one of Claims 1 to 8 and a pharmaceutically acceptable carrier.

10. A compound as claimed in any one of Claims 1 to 8 for use in antagonizing leukotriene action in a mammal.

11. A method of preparing a compound as claimed in Claim 1 that comprises condensing a compound of the formula:

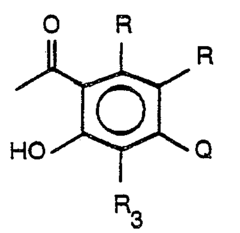

VII ,

where Q is 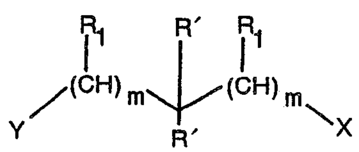 or 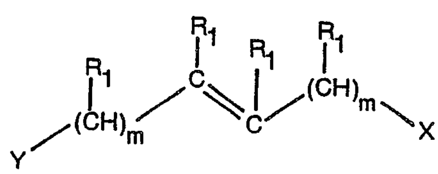

with a compound of the formula 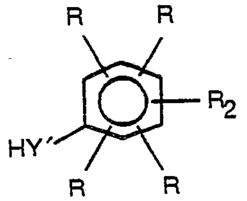

where R, $R_1$, $R_2$, $R_3$, R′, Y′, Y and m have the meanings given in Claim 1, and X is halogen.

12. A method of preparing a compound as claimed in Claim 1 that comprises condensing a compound of the formula

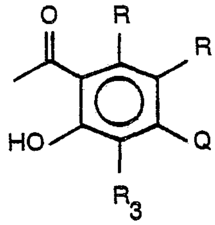

VII,

where Q is 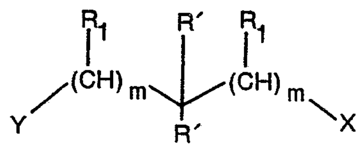 or 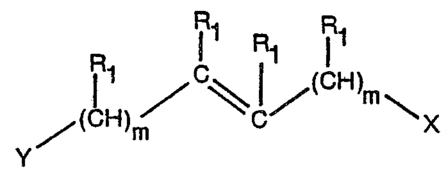

with a compound of the formula 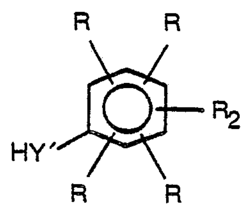

where R, $R_1$, $R_2$, $R_3$, R′, Y′, Y and m have the meaning given in Claim 3, and X is halogen.

13. A method of preparing a compound as claimed in Claim 1 that comprises condensing a compound having one of the following formulae

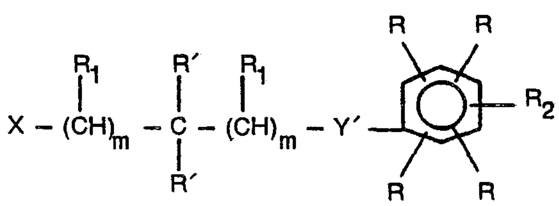

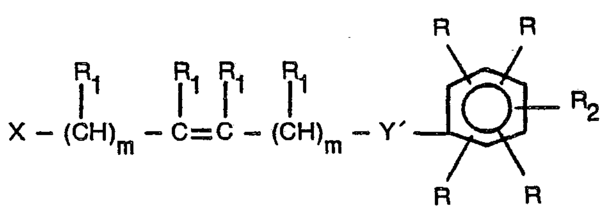

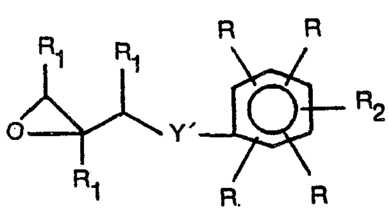

with a compound of the formula 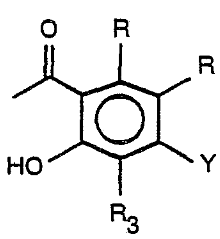,

where $R_1$, $R_2$, $R_3$, R, R′, Y, Y′ and m are as defined in Claim 1, and X is halogen.

14. A method of preparing a compound of Claim 1 that comprises condensing a compound of the formula

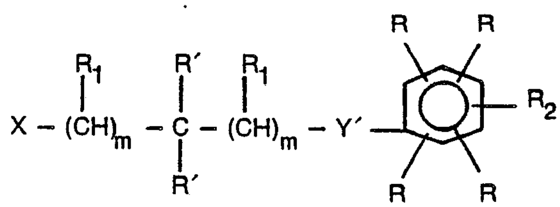

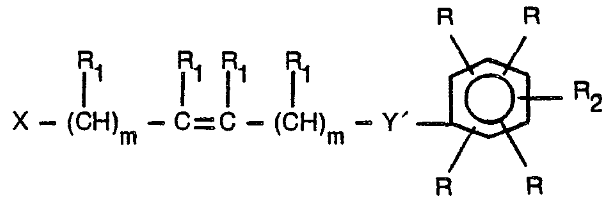

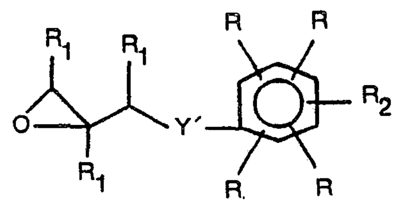

with a compound of the formula 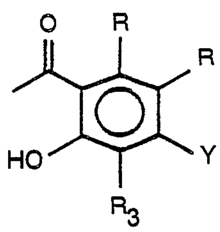 ,

where R, $R_1$, $R_2$, $R_3$, R, Y′, Y and m have the meaning given in Claim 3, and X is halogen.

15. A compound having one of the following formulae

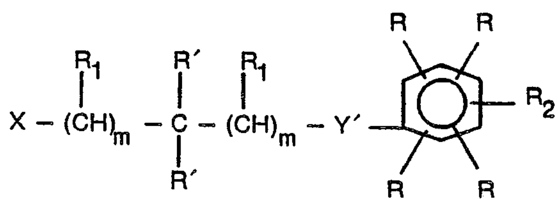

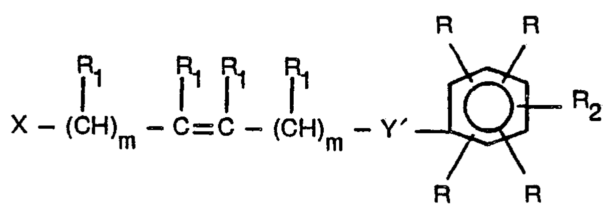

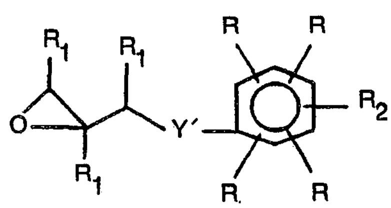

where Y′, R, $R_1$, $R_2$, R′ and m have the meaning given in Claim 1, and X is halogen.

16. A compound having one of the following formulae

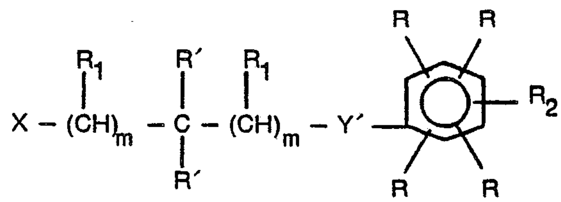

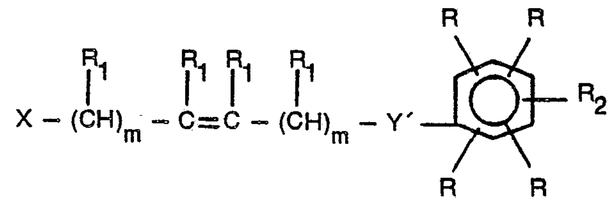

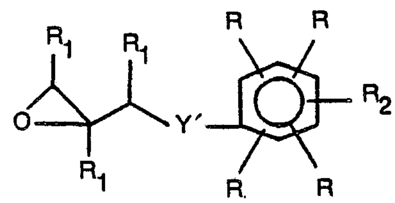

where Y′, R, $R_1$, $R_2$, R′ and m have the meaning given in Claim 3, and X is halogen.

**Claims for the Contracting State: AT**

1. A method of preparing a compound having either of the following formulae

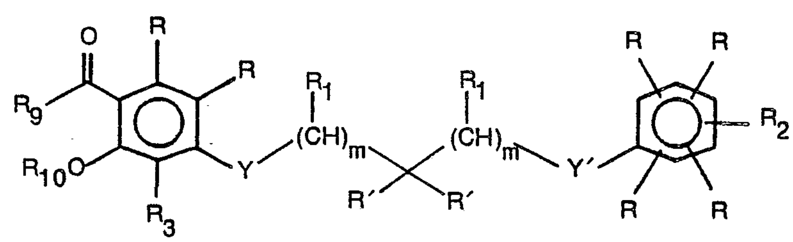

XI

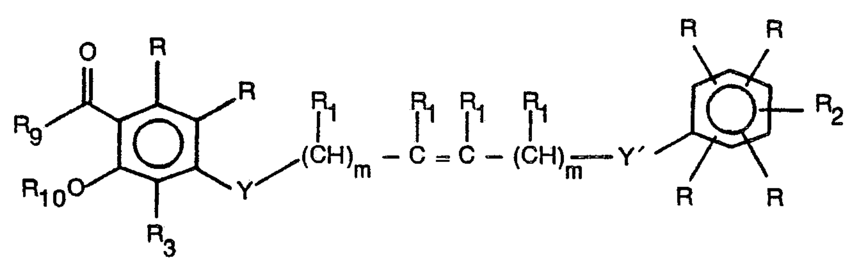

XII

or a compound that is a pharmaceutically acceptable salt or acid-addition salt thereof, that comprises condensing a compound of the formula

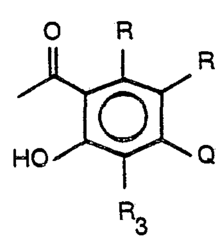

VII,

where Q is 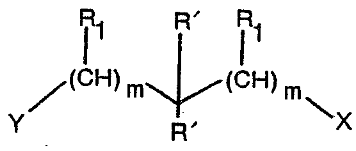 or 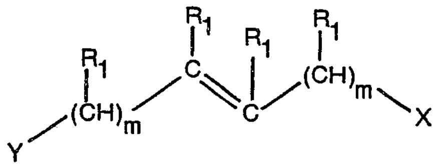

with a compound of the formula 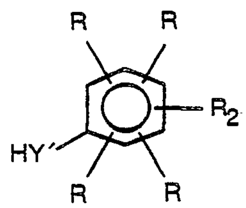

in which formulae X is a halogen;

each R, independently of the others, is H, OH, $C_{1-6}$ alkyl; $C_{2-6}$ alkenyl; trifluoromethyl; $C_{1-6}$ alkoxy; $C_{1-6}$ thioalkyl; phenyl; phenyl substituted by alkyl of 1 to 3 carbon atoms or be halogen; benzyl; phen($C_{2-4}$ alkyl); halogen, $COOR_4$ where $R_4$ is H or $C_{1-6}$ alkyl; CN; trifluoromethylthio; or nitro;

each R′ is independently $R_4$; $OR_4$; $COOR_4$; $N(R_4)_2$; $SR_4$; $CH_2OR_4$; CHO; or together R′ and R′ are O; $CH_2$; or

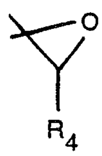 ;

each of Y and Y′, which are the same as or different from one another, is oxygen, sulfur, sulfoxide, sulfone;

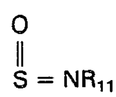

where $R_{11}$ is H, $C_{1-4}$ alkyl, $C_{1-4}$ alkanoyl, phenylsulfonyl or tosyl;

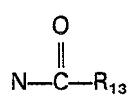

where $R_{13}$ is $C_{1-4}$ alkyl or $C_{1-4}$ alkoxy; N—CN; $CH_2$ or C = O, but with the proviso that Y′ is not oxygen unles Y is oxygen, sulfur, sulfoxide, sulfone or cyanamido and $R_2$ is

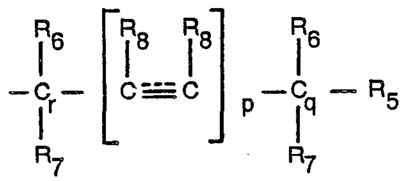 ,

where at least one $R_7$ is OH;

each $R_1$, independently of the others, is hydrogen or $C_{1-3}$ alkyl;

each m, independently of the others, is 0, 1, 2 or 3;

$R_2$ is 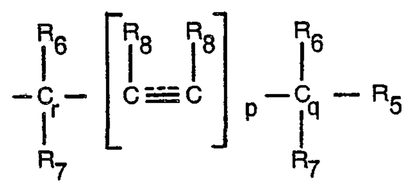 ,

where

each $R_6$, independently of the others, is H or $C_{1-4}$ alkyl;

each $R_7$, independently of the others, is H, OH, or $C_{1-4}$ alkyl;

each $R_8$, independently of the others, is H or $C_{1-4}$ alkyl; and is absent when a triple bond is present;

$R_5$ is $COOR_4$, $CH_2OH$; CHO; tetrazole; hydroxymethylketone; $CONHSO_2R_{14}$; CN; $CON(R_7)_2$; or $COOR_{15}$ where $R_{15}$ is:

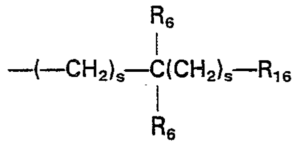

where each s is independently 0—3 and $R_{16}$ is (A) a monocyclic or bicyclic heterocyclic radical containing from 3 to 12 nuclear carbon atoms and 1 or 2 nuclear nitrogen atoms or a nuclear N and a nuclear 5 atom; each ring in the heterocyclic radical being formed of 5 or 6 atoms, or (B) a radical W—$R_{17}$ where W is O, S, or NH and $R_{17}$ contains up to 21 carbon atoms and is a hydrocarbon radical or an acyl radical of an organic acyclic or monocyclic carboxylic acid containing not more than 1 heteroatom in the ring;

each of r and q, independently of the other, is 0 or an integer from 1 to 6, provided that r+q does not exceed 10;

p is 0 or 1;

$R_3$ is $C_{1-6}$ alkyl or $C_{3-6}$ alkenyl;

$R_9$ is OH, $C_{1-6}$ alkyl or $C_{1-6}$ alkoxy;

$R_{10}$ is H; $R_4CO$— or $R_4OCH_2$—;

$R_{14}$ is $C_{1-6}$ alkyl, phenyl, phenyl substituted by $C_{1-3}$ alkyl or alkoxy groups, halogen, hydroxy, haloalkyl, COOH, CN, formyl, $C_{1-6}$ acyl or $C_{1-4}$ perfluoroalkyl; and all carbon chains in all radicals are straight or branched.

2. A method of preparing a compound having formula XI or XII as set forth in Claim 1, in which Y' is oxygen and Y is oxygen, sulfur, sulfoxide, sulfone or cyanamido and $R_2$ is

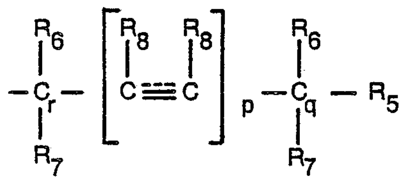 ,

where at least one $R_7$ is OH, the other variables being as defined in Claim 1, that comprises condensing a compound of the formula

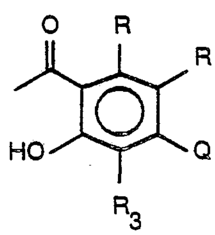 VII,

in which Q is 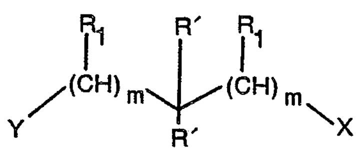 or 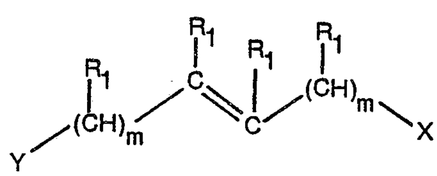

with a compound of the formula 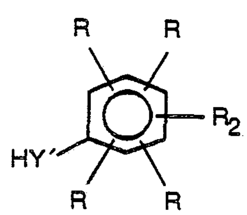

where X is a halogen.

3. A method of preparing a compound having formula XI or XII as set forth in Claim 1, that comprises condensing a compound having one of the following formulae

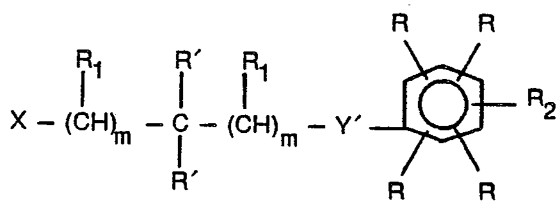

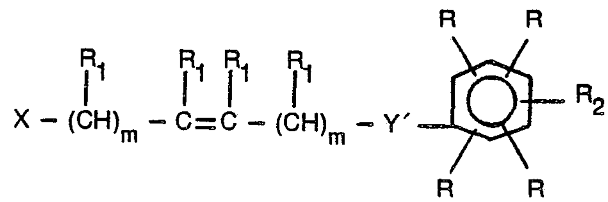

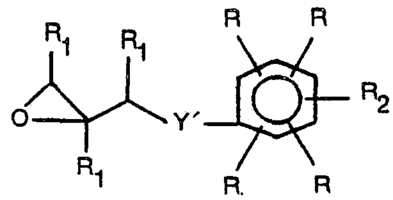

with a compound of the formula 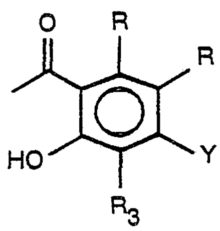 ,

where $R_1$, $R_2$, $R_3$, R, R′, Y, Y′ and m are as defined in Claim 1, and X is halogen.

4. A method of preparing a compound having formula XI or XII as set forth in Claim 1, in which Y′ is oxygen and Y is oxygen, sulfur, sulfoxide, sulfone or cyanamido and $R_2$ is

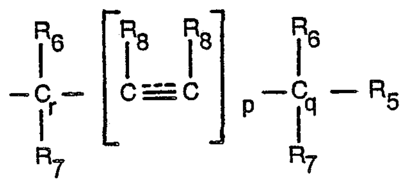 ,

where at least one $R_7$ is OH, the other variables being as defined in Claim 1, that comprises condensing a compound of the formula

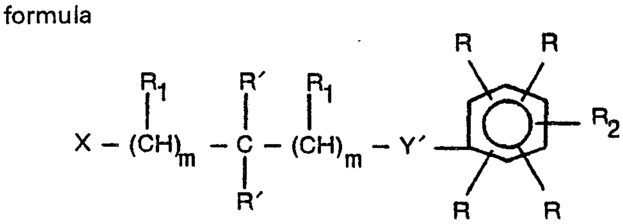

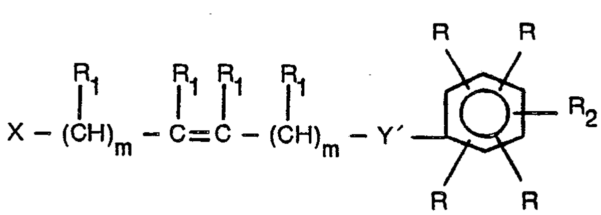

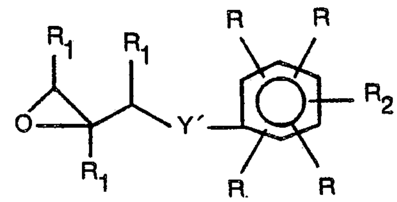

with a compound of the formula 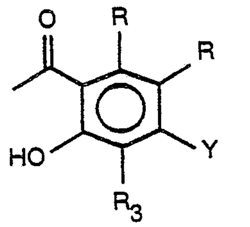 ,

where R, $R_1$, $R_2$, $R_3$, R, Y', Y and m have the meaning given in Claim 3, where X is a halogen.

5. A method as claimed in Claim 1 or 3 as applied to the preparation of a compound in which Y is oxygen and Y' is sulfur, sulfoxide, sulfone, amino or cyanamido.

6. A method as claimed in Claim 2 or as applied to the preparation of a compound in which Y' is oxygen, sulfur, sulfoxide, sulfone or cyanamido and $R_2$ is

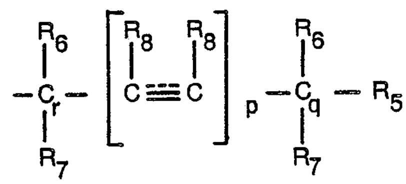 ,

where at least one $R_7$ is OH.

7. A method as claimed in Claim 1 or 3 as applied to the preparation of 4-(3-(4-Acetyl-3-hydroxy-2-propylphenoxy)propylthio)-3-fluorobenzeneacetic acid.

8. A method as claimed in Claim 1 or 2 as applied to the preparation of 4-(3-(4-Acetyl-3-hydroxy-2-propylphenoxy)-propylthio)benzeneacetic acid.

9. A mehod as claimed in Claim 1 or 3 as applied to the preparation of 4-(3-(4-Acetyl-3-hydroxy-2-propylphenoxy)-propylthio)delta-hydroxybenzenebutanol.

10. A method as claimed in Claim 1 or 3 as applied to the preparation of 4-(3-(4-Acetyl-3-hydroxy-2-propyl-phenoxy)-propylsulfonyl)-delta-hydroxy-benzenebutanol.

**Patentansprüche für die Vertragsstaaten: BE CH DE FR GB IT LI LU NL SE**

1. Eine Verbindung einer der folgenden Formeln

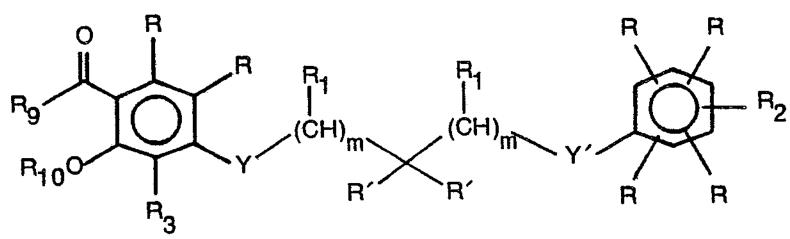

XI

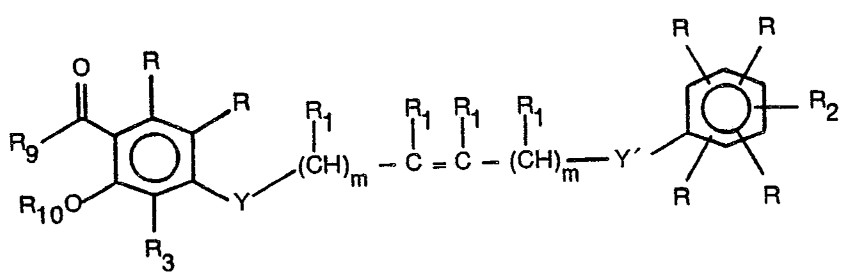

XII

in denen

R unabhängig von den anderen H, OH, $C_{1-6}$-Alkyl; $C_{2-6}$-Alkenyl; Trifluormethyl; $C_{1-6}$-Alkoxy; $C_{1-6}$-Thioalkyl; Phenyl; mit Alkyl von 1 bis 3 Kohlenstoffatomen oder mit Halogen substituiertes Phenyl; Benzyl; Phen($C_{2-4}$-alkyl); Halogen, $COOR_4$, worin $R_4$ H oder $C_{1-6}$-Alkyl; CN; Trifluormethylthio; oder Nitro ist;

jedes R' unabhängig $R_4$; $OR_4$; $COOR_4$; $N(R_4)_2$; $SR_4$; $CH_2OR_4$; CHO ist; oder zusammen R' und R' O; $CH_2$ oder

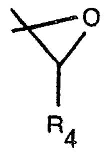 ;

sind;

jedes von Y und Y', die entweder gleich oder unterschiedlich voneinander sind, Sauerstoff, Schwefel, Sulfoxid, Sulfon;

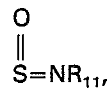

worin $R_{11}$ H, $C_{1-4}$-Alkyl, $C_{1-4}$-Alkanoyl, Phenylsulfonyl oder Tosyl;

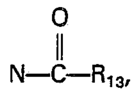

worin $R_{13}$ $C_{1-4}$-Alkyl oder $C_{1-4}$-Alkoxy; N—CN; $CH_2$ oder C=O ist, aber mit der Massgabe, dass Y' nicht Sauerstoff ist, wenn Y Sauerstoff, Schwefel, Sulfoxid, Sulfon oder Cyanamido und $R_2$

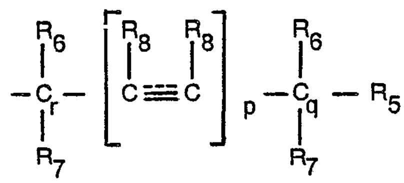 ,

ist, worin mindestens ein $R_7$ OH ist;

jedes $R_1$ unabhängig von den anderen Wasserstoff oder $C_{1-3}$-Alkyl;

jedes m unabhängig von den anderen 0, 1, 2 oder 3;

$R^2$ 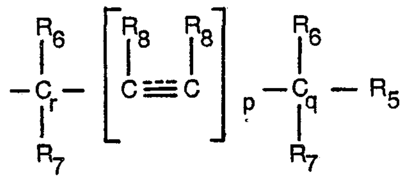 ,

ist,

worin

jedes $R_6$ unabhängig von den anderen H oder $C_{1-4}$-Alkyl;

jedes $R_7$ unabhängig von den anderen H, OH oder $C_{1-4}$-Alkyl;

jedes $R_8$ unabhängig von den anderen H oder $C_{1-4}$-Alkyl; und abwesend ist, wenn eine Dreifachbindung anwesend ist;

$R_5$ $COOR_4$, $CH_2OH$; CHO; Tetrazol; Hydroxymethylketon; $CONHSO_2R_{14}$; CN; $CON(R_7)_2$; oder $COOR_5$, worin $R_{15}$

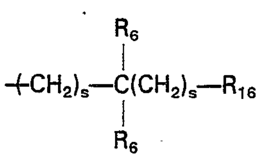

ist, worin jedes s unabhängig 0—3 und $R_{16}$ (A) ein monocyclischer oder bicyclischer heterocyclischer Rest mit 3 bis 12 Kernkohlenstoffatomen und 1 bis 2 Kernstickstoffatomen oder einem Kern N-Atom und einem Kern S-Atom; jeder Ring in dem heterocyclischen Rest von 5 oder 6 Atomen gebildet wird, oder (B) ein Rest W—$R_{17}$, worin W, O, S oder NH ist und $R_{17}$ bis zu 21 Kohlenstoffatomen enthält und ein Kohlenwasserstoffrest oder ein Acylrest einer organischen acyclischen oder monocyclischen Carbonsäure mit nicht mehr als 1 Heteroatom im Ring ist;

jedes von r und q unabhängig von den anderen 0 oder eine ganze Zahl von 1 bis 6 ist, mit der Massgabe, dass r + q nicht 10 überschreitet;

p 0 oder 1;

$R_3$ $C_{1-6}$-Alkyl oder $C_{3-6}$-Alkenyl;

$R_9$ OH, $C_{1-6}$-Alkyl oder $C_{1-6}$-Alkoxy;

$R_{10}$ H, $R_4CO$— oder $R_4OCH_28$;

$R_{14}$ $C_{16}$-Alkyl, Phenyl, mit $C_{1-3}$-Alkyl oder -Alkoxygruppen, Halogen, Hydroxy, Halogenalkyl, COOH, CN, Formyl, $C_{1-6}$-Acyl oder $C_{1-4}$-Perfluoralkyl substituiertes Phenyl; und alle Kohlenstoffketten in allen Resten geradkettig oder verzweigt sind; oder eine Verbindung, die ein pharmazeutisch brauchbares Salz oder Säureadditionssalz davon ist.

2. Eine Verbinding nach Anspruch 1, in der Y Sauerstoff und Y' Schwefel, Sulfoxid, Sulfon, Amino oder Cyanamido ist.

3. Eine Verbindung nach Ansrpuch 1, in der Y' Sauerstoff und Y Sauerstoff, Schwefel, Sulfoxid, Sulfon oder Cyanamido und $R_2$

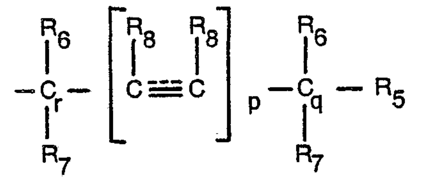

,

ist, worin mindestens ein $R_7$ OH ist.

4. Die folgenden Verbindungen nach Anspruch 1:

4-(3-(4-Acetyl-3-hydroxy-2-propylphenoxy)propylthio)-2,3-dichlorbenzolessigsäure;
4-(3-(4-Acetyl-3-hydroxy-2-propylphenoxy)-2-hydroxypropylthio)-2,3-dichlorbenzolessigsäure;
4-(3-(4-Acetyl-3-hyroxy-2-propylphenopxy)propylthio)-2,3-dichlorbenzolessigsäure-S-oxid;
4-(3-(4-Acetyl-3-hydroxy-2-propylphenoxy)propylsulfonyl)-2,3-dichlorbenzolessigsaüre;
4-(3-(4-Acetyl-3-hydroxy-2-propylphenoxy)-2-hydroxypropylthio)-2,3-dichlorbenzolessigsäure-S-oxid;
4-(3-(4-Acetyl-3-hydroxy-2-propylphenoxy)-2-hydroxypropylsulfonyl)-2,3-dichlorbenzolessigsäure;
4-(3-(4-Acetyl-3-hydroxy-2-propylphenoxy)propylthio)-2-fluorbenzolessigsäure;
4-(3-(4-Acetyl-3-hydroxy-2-propylphenoxy)-2-hydroxypropylthio)-2-fluorbenzolessigsäure;
4-(3-(4-Acetyl-3-hydroxy-2-propylphenoxy)-2-hydroxypropylthio)-2-fluorbenzolessigsärue-S-oxid;
4-(3-(4-Acetyl-3-hydroxy-2-propylphenoxy)-2-hydroxypropylsulfonyl)-2-fluorbenzolessigsäure;
4-(3-(4-Acetyl-3-hydroxy-2-propylphenoxy)-1-propenylsulfonyl)-2-fluorbenzolessigsäure;
4-(3-(4-Acetyl-3-hydroxy-2-propylphenoxy)propylthio)-3-fluorbenzolessigsäure;
4-(3-(4-Acetyl-3-hydroxy-2-propylphenoxy)-2-hydroxypropylthio)-3-fluorbenzolessigsäure;
4-(3-(4-Acetyl-3-hydroxy-2-propylphenoxy)propylthio)-3-chlorbenzolessigsäure;
4-(3-(4-Acetyl-3-hydroxy-2-propylphenoxy)propylthio)-3-chlorbenzolessigsäure-S-oxid;
4-(3-(4-Acetyl-3-hydroxy-2-propylphenoxy)propylsulfonyl)-3-chlorbenzolessigsäure;
4-(3-(4-Acetyl-3-hydroxy-2-propylphenoxy)-2-hydroxypropylthio)-3-chlorbenzolessigsäure;
4-(3-(4-Acetyl-3-hydroxy-2-propylphenoxy)-2-hydroxypropylthio)-3-chlorbenzolessigsäure-S-oxid;
4-(3-(4-Acetyl-3-hydroxy-2-propylphenoxy)-2-hydroxypropylsulfonyl)-3-chlorbenzolessigsäure;
4-(3-(4-Acetyl-3-hydroxy-2-propylphenoxy)propylthio)benzolessigsäure;
4-(3-(4-Acetyl-3-hydroxy-2-propylphenoxy)propylsulfonyl)-benzolessigsäure;
4-(3-(4-Acetyl-3-hydroxy-2-propylphenoxy)-2-methylpropylthio)-benzolessigsäure;
4-(3-(4-Acetyl-3-hydroxy-2-propylphenoxy)-2-hydroxypropylthio)-benzolessigsäure;
4-(3-(4-Acetyl-3-hydroxy-2-propylphenoxy)-2-hydroxypropylthio)-3-fluorbenzolesäure;
4-(3-(4-Acetyl-3-hydroxy-2-propylphenoxy)-2-hydroxypropylthio)-3-fluorbenzosäure-S-oxid;
4-(3-(4-Acetyl-3-hydroxy-2-propylphenoxy)-2-hydroxypropylsulfonyl)-3-fluorbenzoesäure;
4-(3-(4-Acetyl-3-hydroxy-2-propylphenoxy)propylthio-3-fluorbenzoesäure-S-oxid, Methylester;
4-(3-(4-Acetyl-3-hydroxy-2-propylphenoxy)propylsulfonyl)-3-fluorbenzoesäure;
4-(3-(4-Acetyl-3-hydroxy-2-propylphenoxy)propyl)cyanamido)benzolessigsäure;
4-(3-(4-Acetyl-3-hydroxy-2-propylphenoxy)propylthio)delta-hydroxybenzolbutanol;
4-(3-(4-Acetyl-3-hydroxy-2-propylphenoxy)propylsulfonyl)-delta-hydroxybenzolbutanol;
4-(3-(4-Acetyl-3-hydroxy-2-propylphenoxy)propylthio-alpha-hydroxybenzolessigsäure;
4-(3-(4-Acetyl-3-hydroxy-2-propylphenoxy)propylsulfonyl)-alpha-hydroxybenzolessigsäure;
4-(3-(4-Acetyl-3-hydroxy-2-propylphenoxy)propyloxy)-alpha-hydroxybenzolessigsäure;
4-(3-(4-Acetyl-3-hydroxy-2-propylphenoxy)propylthio)-3-brom-alphahydroxybenzolessigsäure;
4-(3-(4-Acetyl-3-hydroxy-2-propylphenoxy)propylthio)-3-fluor-alphahydroxybenzolessigsäure;
2-(3-(4-Acetyl-3-hydroxy-2-propylphenoxy)propyloxy)-alpha-hydroxybenzolessigsäure;
Methyl 4-(4-(3-4-acetyl-3-hydroxy-2-propylphenoxy)propylthio)phenyl)-3-methyl-3(E)-butenoat;
4-(4-(3-(4-Acetyl-4-hydroxy-2-propylphenoxy)propylthio)phenyl)-3-methyl-3(E)-crotonsäure;
Methyl 4-(4-(3-(4-acetyl-3-hydroxy-2-propylphenoxy)propylthio)phenyl-3-methyl-3(Z)-butenoat;

4-(4-(3-(4-Acetyl-3-hydroxyl-2-propylphenoxy)propylthio)phenyl)-3-methyl-3(Z)-crotonsäure.

5. 4-(3-(4-Acetyl-3-hydroxy-2-propylphenoxy)propylthio)-3-fluorbenzolessigsäure.

6. 4-(3-(4-Acetyl-3-hydroxy-2-propylphenoxy)-propylthio)benzolessigsäure.

7. 4-(3-(4-Acetyl-3-hydroxy-2-propylphenoxy)-propylthio)delta-hydroxybenzolessigsäure.

8. 4-(3-(4-Acetyl-3-hydroxy-2-propylphenoxy)propylsulfonyl)delta-hydroxybenzolbutanol.

9. Eine pharmazeutische Zusammensetzung, brauchbar für die Bekämpfung von Leukotrienen in Mammas, insbensondere menschlichen, enthaltend eine wirksame Menge einer Verbindung nach einem der Ansprüche 1 bis 8 und einen pharmazeutisch akzeptablen Träger.

10. Eine Verbindung nach einem der Ansprüche 1 bis 8 für die Verwendung zur Bekämpfung der Leukotrienenwirkung in einer Mamma.

11. Ein Verfahren zur Herstellung einer Verbindung nach Anspruch 1, bei dem eine Verbindung der Formel

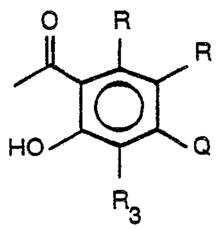

VII,

worin C 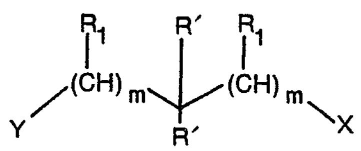 oder 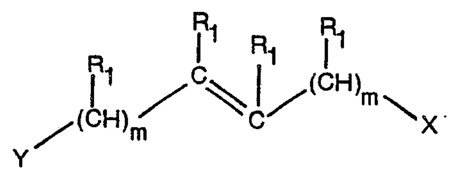

mit einer Verbindung der Formel 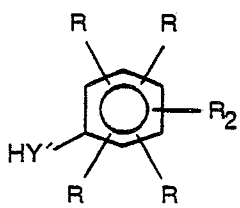

worin R, $R_1$, $R_2$, $R_3$, R', Y', und m de Bedeutungen wie in Anspruch 1 haben und X Halogen ist, kondensiert wird.

12. Ein Verfahren zur Herstellung einer Verbindung nach Anspruch 1, bei dem eine Verbindung der Formel

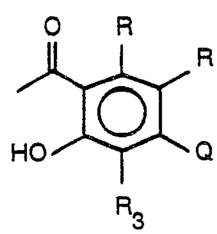

VII,

worin Q 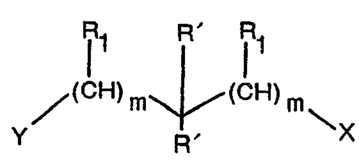 oder 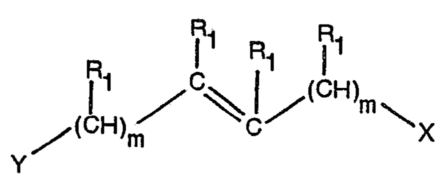

mit einer Verbindung der Formel 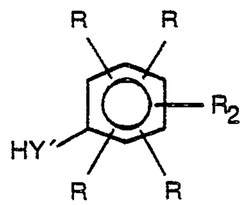

worin R, $R_1$, $R_2$, $R_3$, R', Y', Y und m die Bedeutung wie in Anspruch 3 haben und X Halogen ist, kondensiert wird.

13. Ein Verfahren zur Herstellung einer Verbindung nach Anspruch 1, bei dem eine Verbindung nach einer der folgenden Formeln

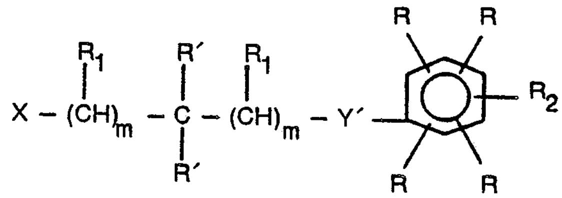

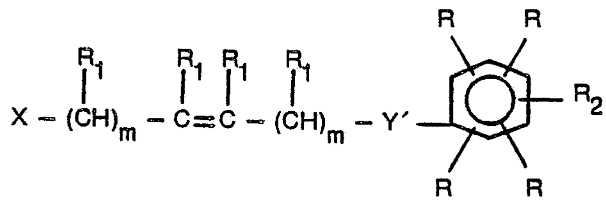

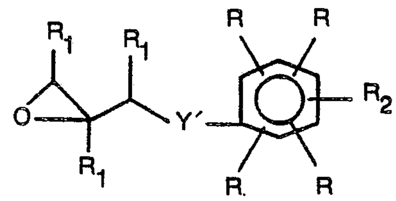

mit einer Verbindung der Formel 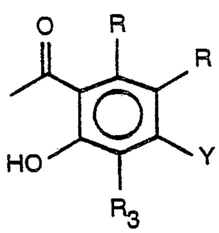 ,

worin R, $R_2$, $R_3$, R, R', Y, Y' und m wie in Anspruch 1 definiert sind und X Halogen ist, kondensiert wird.

14. Ein Verfahren zur Herstellung einer Verbindung nach Anspruch 1, bei dem eine Verbindung der Formel

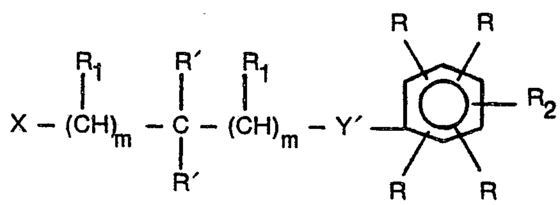

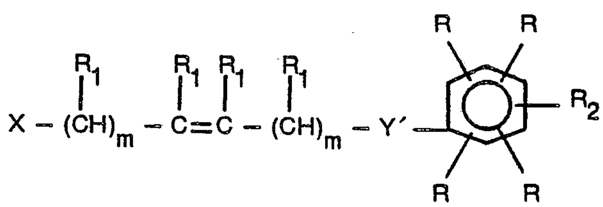

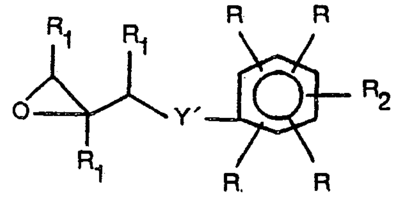

mit einer Verbindung der Formel 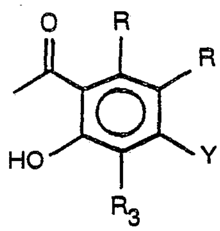 ,

worin R, $R_1$, $R_2$, $R_3$, R, Y', Y und m die Bedeutung wie in Anspruch 3 haben und X Halogen ist, kondensiert wird.

15. Eine Verbindung nach einer der folgenden Formeln

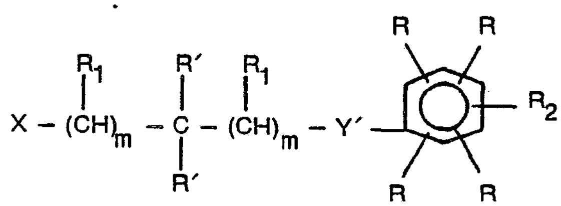

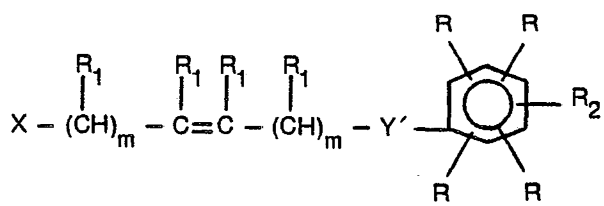

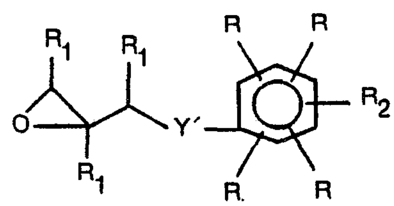

worin Y', R, $R_1$, $R_2$, R' und m die bedeutung wie in Anspruch 1 haben und X Halogen ist.

16. Eine Verbindung nach einer der folgenden Formeln

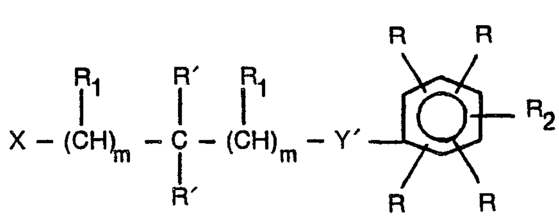

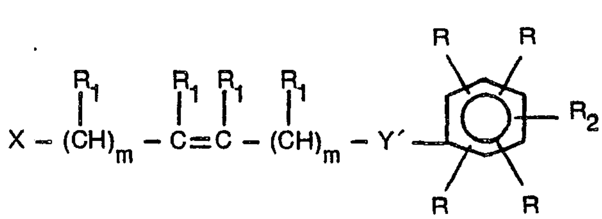

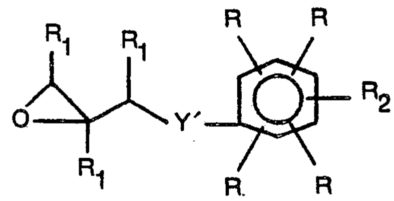

worin Y', R, $R_1$, $R_2$, R' und m die Bedeutung wie in Anspruch 3 haben und X Halogen ist.

**Patentansprüche für den Vertragsstaat: AT**

1. Ein Verfahren zur Herstellung einer Verbindung, die eine der folgenden Formeln aufweist

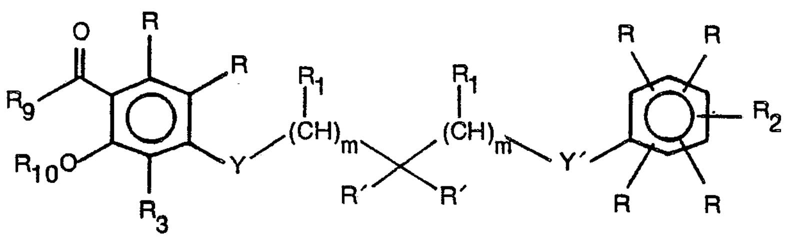

XI

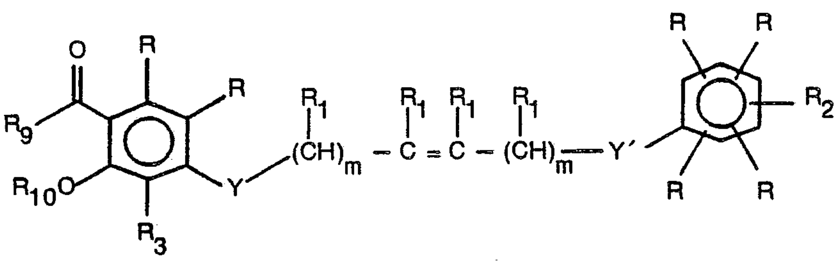

XII

oder einer Verbindung, die ein pharmazeutisch annehmbares Salz oder Säueradditionssalz davon ist, welches das Kondensieren einer Verbindung der Formel

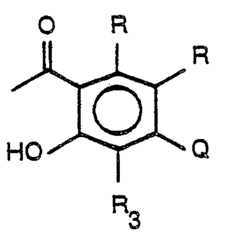

VII,

worin Q 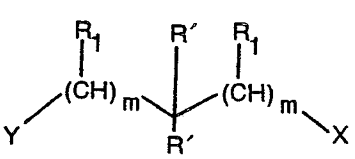 oder 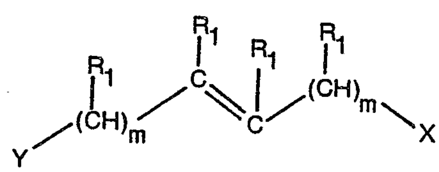

mit einer Verbindung der Formel 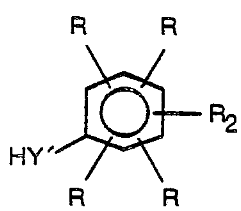

umfaßt, in welchen Formeln X ein Halogen ist;

jeder Rest R, unabhängig von den anderen, H, OH, $C_{1-6}$-Alkyl; $C_{2-6}$-Alkenyl; Trifluormethyl; $C_{1-6}$-

Alkoxy; $C_{1-6}$-Thioalkyl; Phenyl; durch Alkyl mit 1 bis 3 Kohlenstoffatomen oder durch Halogen substituiertes Phenyl; Benzyl; Phen($_{2-4}$-alkyl); Halogen, $COOR_4$, wobei $R_4$ H oder $C_{1-6}$-Alkyl ist; CN; Trifluormethylthio; oder Nitro ist;

jeder Rest R', unabhängig vom anderen, $R_4$; $OR_4$; $COOR_4$; $N(R_4)_2$; $SR_4$; $CH_2OR_4$; oder CHO ist; oder R' und R' gemeinsam O; $CH_2$; oder

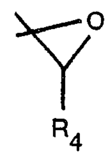 ;

sind;

jeder Rest Y und Y', welche gleich oder voneinander verschieden sind, Sauerstoff, Schwefel, Sulfoxid, Sulfon;

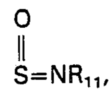

wobei $R_{11}$ H, $C_{1-4}$-Alkyl, $C_{1-4}$-Alkanoyl, Phenylsulfonyl oder Tosyl ist;

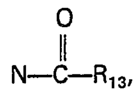

wobei $R_{13}$ $C_{1-4}$-Alkyl oder $C_{1-4}$-Alkoxy ist; N—CN; $CH_2$ oder C=O ist, jedoch mit der Maßgabe, daß Y' nicht Sauerstoff ist, es sei denn, daß Y Sauerstoff, Schwefel, Sulfoxid, Sulfon oder Cyanamido ist, und $R_2$

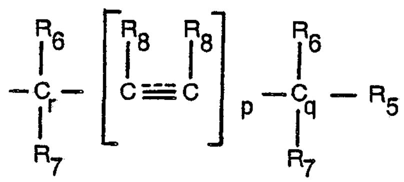 ,

ist, wobei wenigstens ein Rest $R_7$ OH ist;

jedes est $R_1$, unabhängig von den anderen, Wasserstoff oder $C_{1-3}$-Alkylist;

jedes m, unabhängig von den anderen, 0, 1, 2 oder 3 ist;

$R_2$ 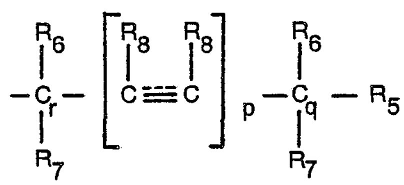 ,

ist, wobei

jedes $R_6$, unabhängig von den anderen, H oder $C_{1-4}$-Alkyl ist;

jedes $R_7$, unabhängig von den anderen, H, OH oder $C_{1-4}$-Alkyl ist;

jedes $R_8$, unabhängig von den anderen, H oder $C_{1-4}$-Alkyl ist; und fehlt, falls eine Dreifachbingung vorliegt;

$R_5$ $COOR_4$, $CH_2OH$; CHO; Tetrazol; Hydroxymethylketon; $CONHSO_2F_{14}$; CN; $CON(R_7)_2$; oder $COOR_{15}$ ist, wobei $R_{15}$:

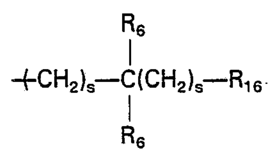

ist, wobei jedes s, unabhängig vom anderen, 0 bis 3 ist, und $R_{16}$ (A) ein monocyclischer oder bicyclischer, heterocyclischer Rest mit 3 bis 12 Kernkohlenstoffatomen und 1 oder 2 Kernstickstoffatomen oder einem Kern-N-und einem Kern-S-Atom ist; wobei jeder Ring in dem heterocyclischen Rest aus 5 oder 6 Atomen gebildet wird, oder (B) ein Rest W—$R_{17}$ ist, wobei W O, S oder NH ist, und $R_{17}$ bis zu 21 Kohlenstoffatome enthält, und ein Kohlenwasserstoffrest oder ein Acylrest einer organischen, acyclischen oder monocyclischen Carbonsäure ist, die nicht mehr als 1 Heteroatom im Ring enthält;

jedes r und 1, unabhängig vom anderen, O oder eine ganze Zahl von 1 bis 6 mit der Maßgabe ist, daß r + q den Wert 10 nicht überschreitet,

p O oder 1 ist;

$R_3$ $C_{1-6}$-Alkyl oder $C_{3-6}$-Alkenyl ist;

$R_9$ OH, $C_{1-6}$-Alkyl oder $C_{1-6}$-Alkoxy ist;

$R_{10}$ H, $R_4CO$— oder $R_4OCH_2$— ist;

$R_{14}$ $C_{1-6}$-Alkyl, Phenyl, durch $C_{1-3}$-Alkyl-oder Alkoxy-gruppen substituiertes Phenyl, Halogen, Hydroxy, Halogenalkyl, COOH, CN, Formyl, $C_{1-6}$-Acyl oder $C_{1-4}$-Perfluoralkyl ist; und alle Kohlenstoffketten in allen Resten gerade oder verzweigt sind.

2. Ein Verfahren zur Herstellung einer Verbindung der Formel XI oder XII, wie in Anspruch 1 angegeben, in welcher Y' Sauerstoff ist und Y Sauerstoff, Schwefel, Sulfoxid, Sulfon oder Cyanamido ist, und $R_2$

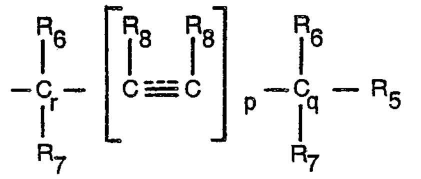 ,

ist, wobei wenigstens ein Rest $R_7$ OH ist, und die anderen Variablen wie in Anspruch 1 definiert sind, umfassend das Kondensieren einer Verbindung der Formel

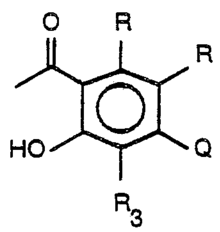 VII,

in welcher Q 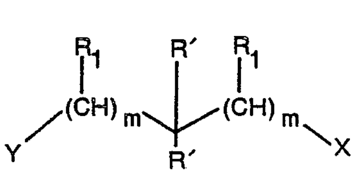 oder 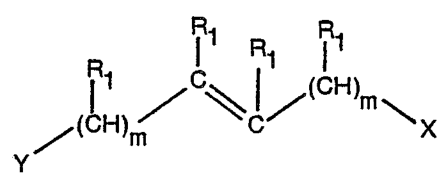

ist, mit einer Verbindung der Formel 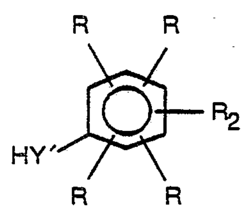

worin X ein Halogen ist.

3. Ein Verfahren zur Herstellung einer Verbindung mit der Formel XI oder XII, wie in Anspruch 1 angegeben, umfassend das Kondensieren einer Verbindung, die eine der folgenden Formeln aufweist:

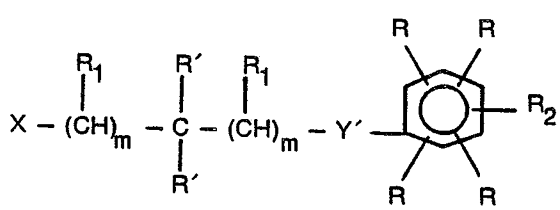

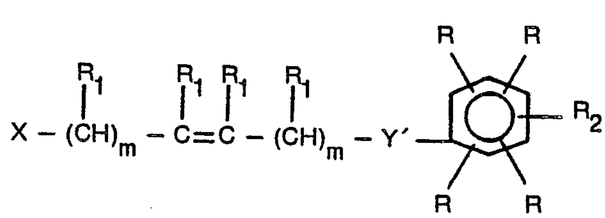

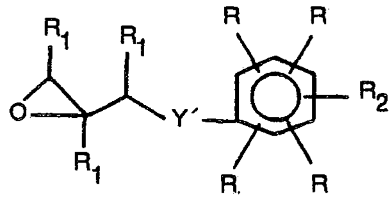

mit einer Verbindung der Formel

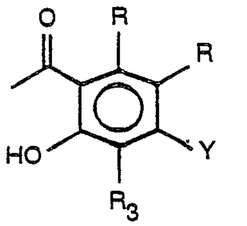

,

worin $R_1$, $R_2$, $R_3$, R, R', Y, Y' und m wie in Anspruch 1 definiert sind, und X Halogen ist.

4. Ein Verfahren zur Herstellung einer Verbindung mit der Formel XI oder XII, wie, in Anspruch 1 angegeben, in welcher Y' Sauerstoff ist, und Y Sauerstoff, Schwefel, Sulfoxid, Sulfon oder Cyanamido ist, und $R_2$

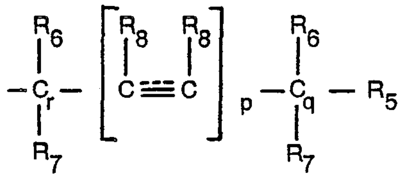

,

ist, wobei wenigstens ein Rest $R_7$ OH ist, und die anderen Variablen wie in Anspruch 1 definiert sind, umfassend das Kondensieren einer Verbindung der Formel:

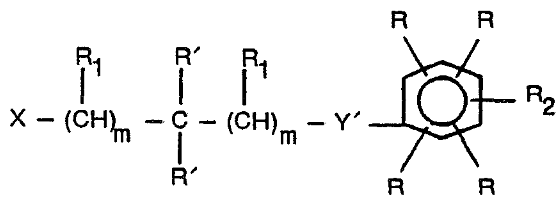

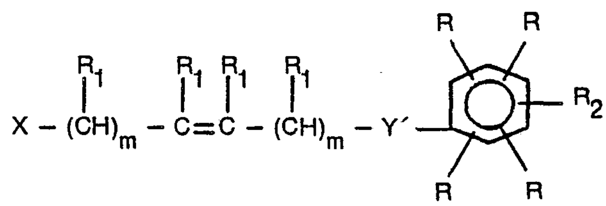

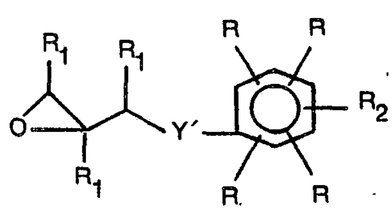

mit einer Verbindung der Formel

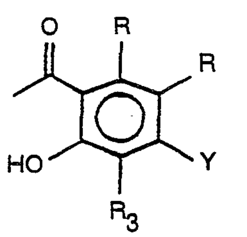

,

worin $R_1$, $R_2$, $R_3$, R, R', Y, Y' und m die in Anspruch 3 angegebene Bedeutung haben; wobei X ein Halogen ist.

5. Ein Verfahren wie in Anspruch 1 oder 3 beansprucht, angewendet bei der Herstellung einer Verbindung, in welcher Y Sauerstoff ist, und Y' Schwefel, Sulfoxid, Sulfon, Amino oder Cyanamido ist.

6. Ein Verfahren wie in Anspruch 2 oder 3 beansprucht, angewendet bei der Herstellung einer Verbindung, in welcher Y' Sauerstoff ist und Y Sauerstoff, Schwefel, Sulfoxid, Sulfon oder Cyanamido ist, und $R_2$

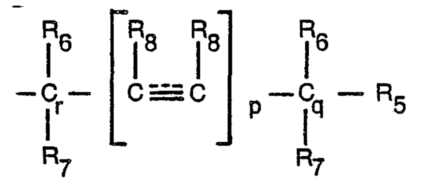 ,

ist, wobei wenigstens ein Rest $R_7$ OH ist.

7. Ein Verfahren wie in Anspruch 1 oder 3 beansprucht, angewendet bei der Herstellung von 4-(3-(4-Acetyl-3-hydroxy-2-propylphenoxy)propylthio)-3-fluorbenzolessigsäure.

8. Ein Verfahren wie in Anspruch 1 oder 2 beansprucht, angewendet bei der Herstellung von 4-(3-(4-Acetyl-3-hydroxy-2-propylphenoxy)-propylthio)benzolessigsäure.

9. Ein Verfahren wie in Anspruch 1 oder 3 beansprucht, angewendet bei der Herstellung von 4-(3-(4-Acetyl-3-hydroxy-2-propylphenoxy)-propylthio)delta-hydroxybenzolbutanol.

10. Ein Verfahren wie in Anspruch 1 oder 3 beansprucht, angewendet bei der Herstellung von 4-(3-(4-Acetyl-3-hydroxy-2-propyl-phenoxy)-propylsulfonyl)delta-hydroxybenzolbutanol.

**Revendications pour les Etats contractants: BE CH DE FR GB IT LI LU NL SE**

1. Composé ayant l'une quelconque des formules suivantes:

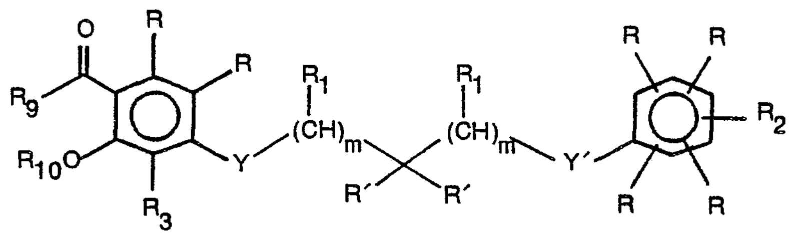 XI

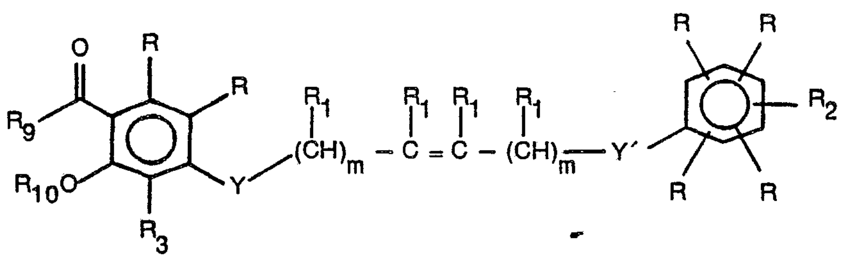 XII

dans lesquelles

chaque R est, indépendament des autres, H, OH, un groupe alkyle en $C_{1-6}$; un groupe alcényle en $C_{2-6}$; un groupe trifluorméthyle; un groupe alcoxy en $C_{1-6}$; un groupe thioalkyle en $C_{1-6}$; un groupe phényle; un groupe phényle substitué par un groupe alkyle de 1 à 3 atomes de carbone ou par un halogène; un groupe benzyle; un groupe phényl(alkyle en $C_{2-4}$); un halogène, $COOR_4$ dans lequel $R_4$ est H ou un groupe alkyle en $C_{1-6}$; CN; un groupe trifluorméthylthio; ou un groupe nitro;

chaque R' est indépendamment $R_4$; $OR_4$; $COOR_4$; $N(R_4)_2$; $SR_4$; $CH_2OR_4$; ou R' et R' sont ensemble O; $CH_2$; ou

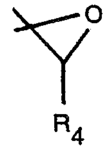 ;

chaque Y et Y', qui sont identiques ou différents l'un de l'autre, est l'oxygène, le soufre, le sulfoxyde, une sulfone;

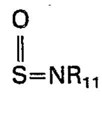

dans laquel $R_{11}$ est H, un groupe alkyle en $C_{1-4}$, un groupe alcanoyle en $C_{1-4}$, un groupe phénylsulfonyle ou tosyle;

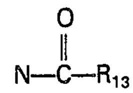

dans lequel $R_{13}$ est un groupe alkyle en $C_{1-4}$ ou un groupe alcoxy en $C_{1-4}$; N—CN; $CH_2$ ou C=O, mais à la condition que Y' ne soit pas l'oxygène à moins que Y ne soit l'oxygène, le soufre, le sulfoxyde, une sulfone ou un cyanamido et que $R_2$ ne soit

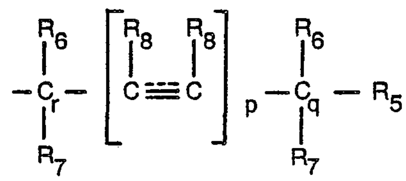 ,

dans lequel au moins un $R_7$ est OH;

chaque $R_1$ est, indépendamment des autres, l'hydrogène ou un groupe alkyle en $C_{1-3}$.

chaque m est, indépendamment des autres, 0, 1, 2 ou 3;

$R_2$ est 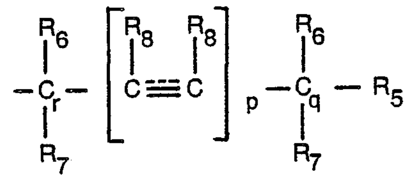 ,

dans lequel

chaque $R_6$ est, indépendamment des autres, H ou un groupe alkyle en $C_{1-4}$;

chaque $R_7$ est, indépendamment des autres, H, OH ou un groupe alkyle en $C_{1-4}$;

chaque $R_8$ est, indépendamment des autres, H ou un groupe alkyle en $C_{1-4}$; et est absent lorsqu'une triple liaison est présente;

$R_5$ est $COOR_4$; $CH_2OH$; un groupe tétrazole; hydroxyméthylcétone; $CONHSO_2R_{14}$; CN; $CON(R_7)_2$; ou $COOR_{15}$ dans lequel $R_{15}$ est

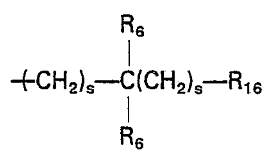

où chaque s est indépendamment 0—3 et $R_{16}$ est (A) un radical hétérocyclique monocyclique ou bicyclique contenant de 3 à 12 atomes de carbone dans le noyau et de 1 à 2 atomes d'azote dans le noyau ou un atome de N dans le noyau et un atome de S dans le noyau; chaque cycle du radical hétérocyclique étant formé de 5 ou 6 atomes, ou (B) un radical W—$R_{17}$ où W est O, S ou NH et $R_{17}$ contient jusqu'à 21 atomes de carbone et est un radical d'hydrocarbure ou un radical acyle d'un acide carboxylique organique acyclique ou monocyclique ne contenant pas plus de l hétéroatome dans le cycle;

chaque r et q est, indépendamment l'un de l'autre, 0 ou un nombre entier de 1 à 6, à condition que r + q ne dépasse pas 10;

p est 0 ou 1;

$R_3$ est un groupe alkyle en $C_{1-6}$ ou alcényle en $C_{3-6}$;

$R_9$ est OH, un groupe alkyle en $C_{1-6}$ ou alcoxy en $C_{16}$;

$R_{10}$ est H; $R_4CO$— ou $R_4OCH_2$—;

$R_{14}$ est un groupe alkyle en $C_{1-6}$, phényle, phényle substitué par des groupes alkyle ou alcoxy en $C_{1-3}$, halogène, hydroxy, halogénoalkyle, COOH, CN, formyle, acyle en $C_{1-6}$ ou perfluoroalkle en $C_{1-4}$; et toutes les chaînes carbonées de tous les radicaux sont droites ou ramifiées; ou compose qui est un de leurs sels pharmaceutiquement acceptables ou de leurs sels d'addition avec un acide.

2. Composé selon la revendication 1, dans lequel Y est l'oxygène et Y' est le soufre, un groupe sulfoxyde, sulfone, amino ou cyanamido.

3. Composé selon la reventication 1, dans lequel Y' est l'oxygène et Y est l'oxygène, le soufre, un groupe sulfoxyde, sulfone ou cyanamido et $R_2$ est

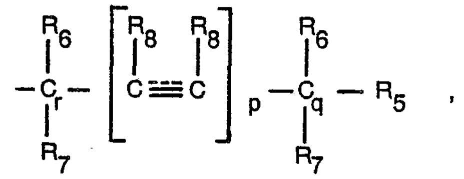

dans lequel au moins un $R_7$ est OH.

4. Composés selon la revendication 1 suivants:

Acide 4-(3-(4-acétyl-3-hydroxy-2-propylphénoxy)-propylthio)-2,3-dichlorobenzène-acétique

Acide 4-(3-(4-acétyl-3-hydroxy-2-propylphénoxy)-2-hydroxypropylthio)-2,3-dichlorobenzène-acétique

S-Oxyde d'acide 4-(3-(4-acétyl-3-hydroxy-2-propylphénoxy)propylthio)-2,3-dichlorobenzéne-acétique

Acide 4-(3-(4-acétyl-3-hydroxy-2-propylphénoxy)-propylsulfonyl)-2,3-dichlorobenzène-acétique

S-Oxyde d'acide 4-(3-(4-acétyl-3-hydroxy-2-propylphénoxy)-2-hydroxypropylthio)2,3-dichlorobenzène-acétique

Acide 4-(3-(4-acétyl-3-hydroxy-2-propylphénoxy)-2-hydroxypropylsulfonyl)-2,3-dichlorobenzène-acétique

Acide 4-(3-(4-acétyl-3-hydroxy-2-propylphénoxy)-propylthio)-2-fluorobenzène-acétique

Acide 4-(3-(4-acétyl-3-hydroxy-2-propylphénoxy)-2-hydroxypropylthio)-2-fluorobenzène-acétique

S-Oxyde d'acide 4-(3-(4-acétyl-3-hydroxy-2-propylphénoxy)-2-hydroxypropylthio)-2-hydroxypropylthio)-2-fluorobenzène-acétique

Acide 4-(3-(4-acétyl-3-hydroxy-2-propylphénoxy)-2-hydroxypropylsulfonyl)-2-fluorobenzène-acétique

Acide 4-(3-(4-acétyl-3-hydroxy-2-propylphénoxy)-1-propénylsulfonyl)-2-fluorobenzène-acétique

Acide 4-(3-(4-acétyl-3-hydroxy-2-propylphénoxy)-propylthio)-3-fluorobenzène-acètique

Acide 4-(3-(4-acétyl-3-hydroxy-2-propylphénoxy)-2-hydroxypropylthio)-3-fluorobenzène-acétique

Acide 4-(3-(4-acétyl-3-hydroxy-2-propylphénoxy)-propylthio)-3-chlorobenzène-acétique

S-Oxyde d'acide 4-(3-(4-acétyl-3-hydroxy-2-propylphénoxy)propylthio)-3-chlorobenzène-acétique

Acide 4-(3-(4-acétyl-3-hydroxy-2-propylphénoxy)-propylsulfonyl)-3-chlorobenzène-acétique

Acide 4-(3-(4-acétyl-3-hydroxy-2-propylphénoxy)-2-hydroxypropylthio)-3-chlorobenzène-acétique

S-Oxyde d'acide 4-(3-(4-acétyl-3-hydroxy-2-propylphénoxy)-2-hydroxypropylthio)-3-chlorobenzène-acétique

Acide 4-(3-(4-acétyl-3-hydroxy-2-propylphénoxy)-2-hydroxypropylsulfonyl)-3-chlorobenzène-acétique

Acide 4-(3-(4-acétyl-3-hydroxy-2-propylphénoxy)propylthio)benzène-acétique

Acide 4-(3-(4-acétyl-3-hydroxy-2-propylphénoxy)-propylsulfonyl)benzène-acétique

Acide 4-(3-(4-acétyl-3-hydroxy-2-propylphénoxy)-2-méthylpropylthio)benzène-acétique

Acide 4-(3-(4-acétyl-3-hydroxy-2-propylphénoxy)-2-hydroxypropylthio)benzène-acétique

Acide 4-(3-(4-acétyl-3-hydroxy-2-propylphénoxy)-2-hydroxypropylthio)-3-fluorobenzoïque

S-Oxyde d'acide 4-(3-(4-acétyl-3-hydroxy-2-propylphénoxy)-2-hydroxypropylthio)-3-fluorobenzoïque

Acide 4-(3-(4-acétyl-3-hydroxy-2-propylphénoxy)-2-hydroxypropylsulfonyl)-3-fluorobenzoïque

Ester méthylique du S-oxyde d'acide 4-(3-(4-acétyl-3-hydroxy-2-propylphénoxy)propylthio)-3-fluorobenzoïque

Acide 4-(3-(4-acétyl-3-hydroxy-2-propylphénoxy)-propylsulfonyl)-3-fluorobenzoïque

Acide 4-(3-(4-acétyl-3-hydroxy-2-propylphénoxy)-propylcyanamido)benzène-acétique

4-(3-(4-Acétyl-3-hydroxy-3-propylphénoxy)phenylthio)-delta-hydroxybenzène-butanol

4-(3-(4-Acétyl-3-hydroxy-2-propylphénoxy)propylsulfonyl)-delta-hydroxybenzène-butanol

Acide 4-(3-(4-acétyl-3-hydroxy-2-propylphénoxy)-propylthio)-alpha-hydroxybenzène-acétique

Acide 4-(3-(4-acétyl-3-hydroxy-2-propylphénoxy)-propylsulfonyl)-alpha-hydroxybenzène-acétique

Acide 4-(3-(4-acétyl-3-hydroxy-2-propylphénoxy)-propyloxy)-alpha-hydroxybenzène-acétique

Acide 4-(3-(4-acétyl-3-hydroxy-2-propylphénoxy)-propylthio)-3-bromo-alpha-hydroxybenzène-acétique

Acide 4-(3-(4-acétyl-3-hydroxy-2-propylphénoxy)-propylthio)-3-fluoro-alpha-hydroxybenzène-acétique

Acide 2-(3-(4-acétyl-3-hydroxy-2-propylphénoxy)-propyloxy)-alpha-hydroxybenzène-acétique

4-(4-(3-(4-Acétyl-3-hydroxy-2-propylphénoxy)propylthio)phényl-3-methyl-3(E)-buténoate de méthyle

Acide 4-(4-(3-(4-acétyl-4-hydroxy-2-propylphénoxy)propylthio)phényl)-3-méthyl-3(E)-buténoïque

4-(4-(3-(4-Acétyl-3-hydroxy-2-propylphénoxy)propylthio)phényl)-3-méthyl-3(Z)-buténoate de méthyle

Acide 4-(4-(3-(4-acétyl-3-hydroxy-2-propylphénoxy)propylthio)phényl)-3-méthyl-3(Z)-buténoïque.

5. Acide 4-(3-(4-acétyl-3-hydroxy-2-propylphénoxy)propylthio)-3-fluoro-benzène-acétique.

6. Acide 4-(3-(4-acétyl-3-hydroxy-2-propylphénoxy)propylthio)benzène-acétique.

7. 4-(3-(4-Acétyl-3-hydroxy-2-propylphénoxy)propylthio)-delta-hydroxybenzène-butanol.

8. 4-(3-(4-Acétyl-3-hydroxy-2-propylphénoxy)propylsulfonyl)-delta-hydroxybenzène-butanol.

9. Composition pharmaceutique utilisable pour antagoniser les leucotriènes chez les mammifères, en

particulier chez l'homme, contenant une quantité efficace d'un composé selon l'une quelconque des revendications 1 à 8 et un véhicule pharmaceutiquement acceptable.

10. Composé selon l'une quelconque des revendications 1 à 8, utilisable pour antagoniser l'action des leucotriènes chez un mammifère.

11. Procédé de préparation d'un composé selon la revendication 1, qui comprend la condensation d'un composé de formule

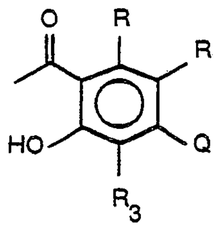

VII ,

où Q est 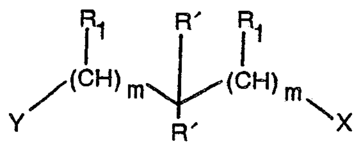 ou 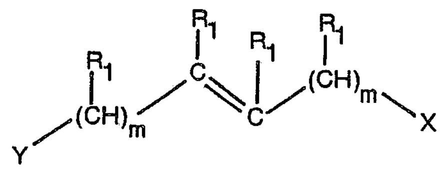

avec un composé de formule 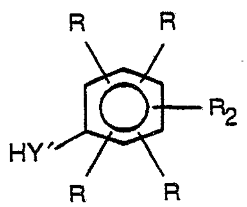

dans laquelle R, $R_1$, $R_2$, $R_3$, R', Y', Y et m ont les significations données dans la revendication 1 et X est un halogène.

12. Procédé de préparation d'un composé selon la revendication 1, qui comprend la condensation d'un composé de formule

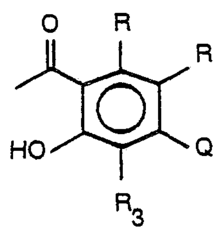

VII ,

où Q est 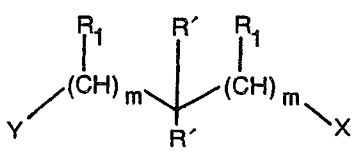 ou 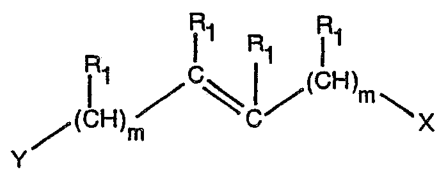

avec un composé de formule 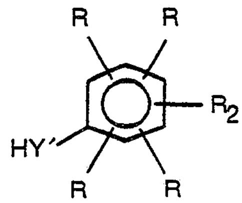

dans laquelle R, $R_1$, $R_2$, $R_3$, R', Y', Y et m ont les significations données dans la revendication 1 et X est un halogène.

13. Procédé de preparation d'un composé selon la revendication 1, qui comprend la condensation d'un composé ayant l'une des formules suivantes

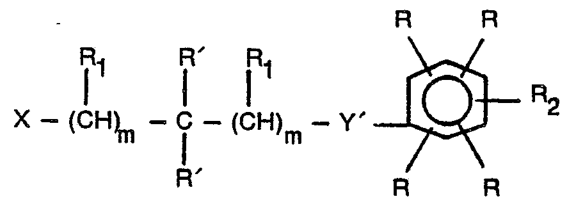

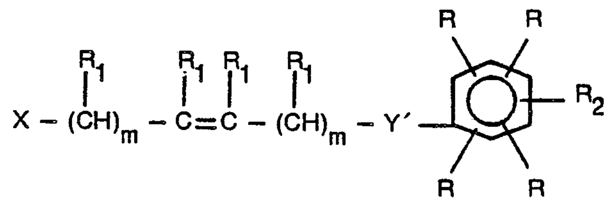

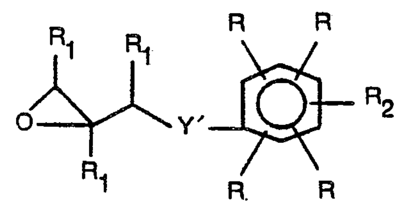

avec un composé de formule 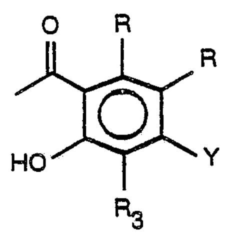 ,

dans laquelle $R_1$, $R_2$, $R_3$, R, R', Y, Y' et m sont tels que définis dans la revendication 1 et X est un halogène.

14. Procédé de préparation d'un composé selon la revendication 1, qui comprend la condensation d'un composé ayant l'une des formules suivantes

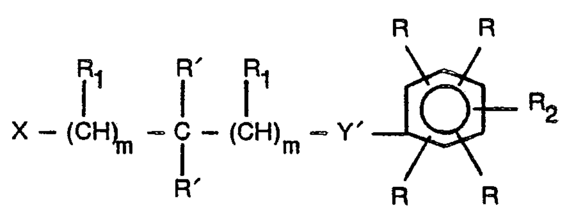

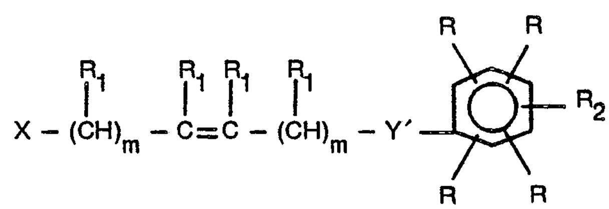

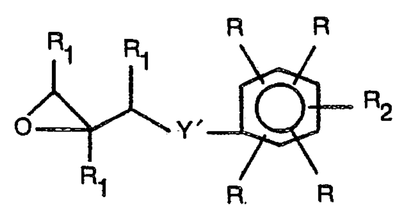

avec un composé de formule

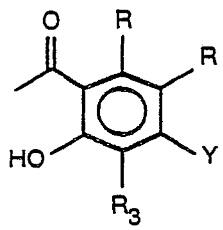

,

dans laquelle R, $R_1$, $R_2$, $R_3$, R, Y, Y' et m ont la signification donnée dans la revendication 3 et X est un halogène.

15. Composé ayant l'une des formules suivantes

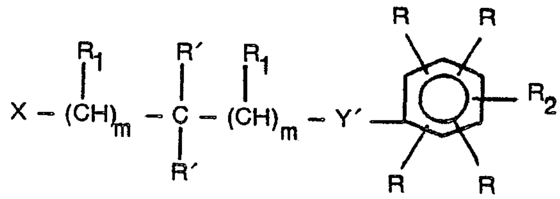

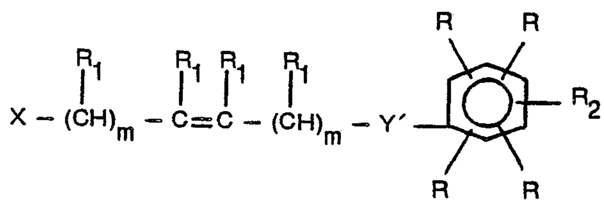

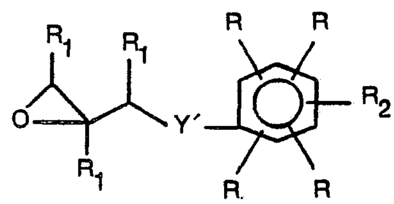

dans lesquelles Y', R, $R_1$, $R_2$, R' et m ont la signification donnée dans la revendication 1, et X est un halogène.

16. Composé ayant l'une des formules suivantes

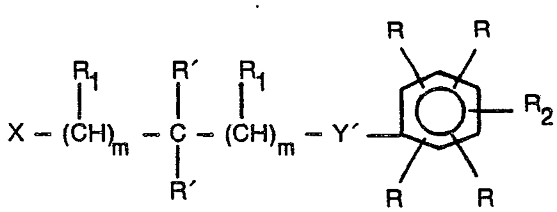

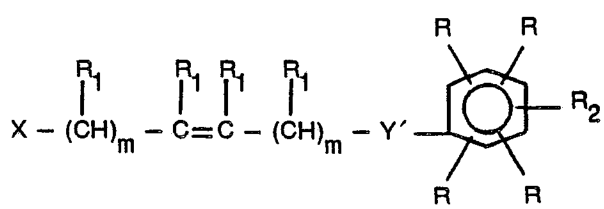

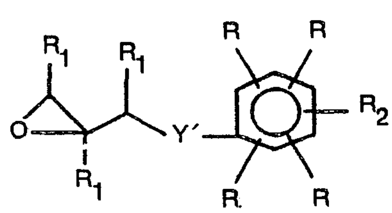

dans lesquelles Y', R, $R_1$, $R_2$, R' et m ont la signification donnée dans la revendication 3, et X est un halogène.

**Revendications pour l'Etat contractant: AT**

1. Procédé de préparation d'un composé ayant l'une des formules suivantes

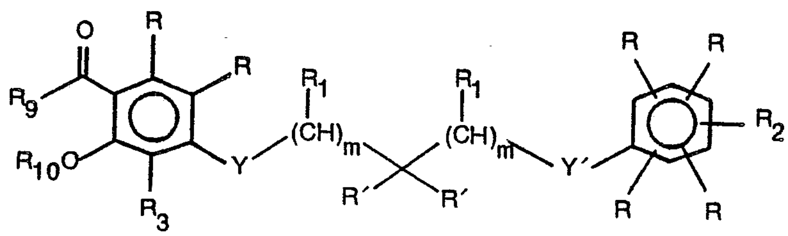

XI

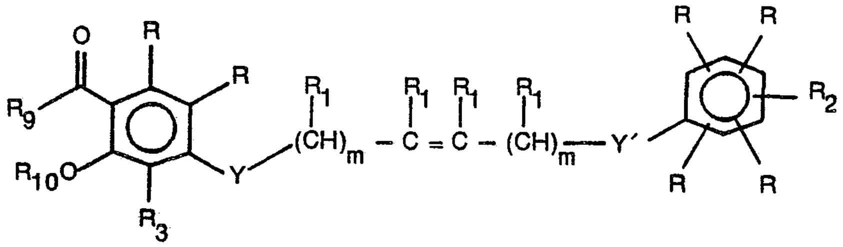

XII

ou d'un composé qui est un de ses sels pharmaceutiquement acceptables ou un de ses sels d'addition avec un acide, qui comprend la condensation d'un composé de formule

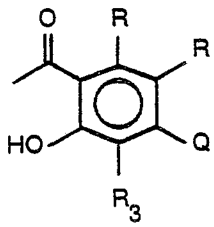

VII,

où Q est

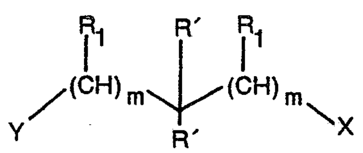

ou

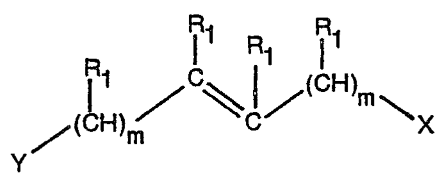

avec un composé de formule

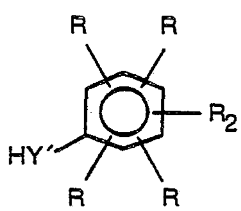

formules dans lesquelles X est un halogène;

chaque R est, indépendament des autres, H, OH, un groupe alkyle en $C_{1-6}$; un groupe alcényle en $C_{2-6}$; un groupe trifluorométhyle; un groupe alcoxy en $C_{1-6}$; un groupe thioalkyle en $C_{1-6}$; un groupe phényle; un groupe phényle substitué par un groupe alkyle de 1 à 3 atomes de carbone ou per un halogène; un groupe benzyle; un groupe phényl(alkyle en $C_{2-4}$); un halogène, $COOR_4$ dans lequel $R_4$ est H ou un groupe alkyle en $C_{1-6}$; CN; un groupe trifluorométhylthio; ou un groupe nitro;

chaque R' est indépendamment $R_4$; $OR_4$; $COOR_4$;

chaque R' est indépendamment $R_4$; $OR_4$; $COOR_4$; $N(R_4)_2$; $SR_4$; $CH_2OR_4$; CHO; ou R' et R' sont ensemble 0; $CH_2$; ou

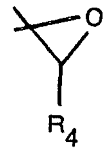 ;

chaque Y et Y', qui sont identiques ou différents l'un de l'autre, est l'oxygène, le soufre, le sulfoxyde, une sulfone;

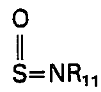

dans lequel $R_{11}$ est H, un groupe alkyle en $C_{1-4}$, un groupe alcanoyle en $C_{1-4}$, un groupe phenylsylfonyle ou tosyle;

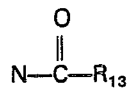

dans lequel $R_{13}$ est un groupe alkyle en $C_{1-4}$ ou un groupe alcoxy en $C_{1-4}$; N—CN; $CH_2$ ou C=O, mais à la condition que Y' ne soit pas l'oxygène à moins que Y ne soit l'oxygène, le soufre, le sulfoxyde, une sulfone ou un cyanamido et que $R_2$ ne soit

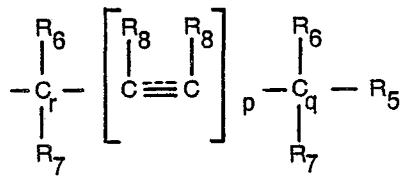 ,

dans lequel au moins un $R_7$ est OH;

$R_2$ est 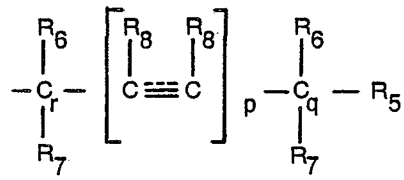 ,

dans lequel

chaque $R_6$ est, indépendamment des autres, H ou un groupe alkyle en $C_{1-4}$;

chaque $R_7$ est, indépendamment des autres, H, OH ou un groupe alkyle en $C_{1-4}$;

chaque $R_8$ est, indépendamment des autres, H ou un groupe alkyle en $C_{1-4}$; et est lorsqu'une triple liaison est présente;

$R_5$ est $COOR_4$; $CH_2OH$; CHO; un groupe tétrazole; hydroxyméthylcétone; $CONHSO_2R_{14}$; CN; $CON(R_7)_2$; ou $COOR_{15}$ dans lequel $R_{15}$ est

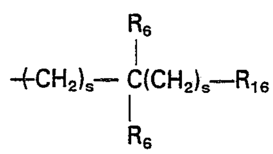

où chaque s est indépendamment 0—3 et $R_{16}$ est (A) un radical hétérocyclique ou bicyclique contenant de 3 à 12 atomes de carbone dans le noyau et de 1 à 2 atomes d'azote dans le noyau ou un atome de N dans le noyau et un atome de S dans le noyau; chaque cyle du radical hétérocyclique étant formé de 5 ou 6 atomes, ou (B) un radical W—$R_{17}$ où W est O, S ou NH et $R_{17}$ contient jusqu'à 21 atomes de carbone et est un radical d'hydrocarbure ou un radical acyle d'un acide carboxylique organique acyclique ou monocyclique ne contenant pas plus de 1 hétéroatome dans le cycle;

chaque r et 1 est, indépendamment l'un de l'autre, 0 ou un nombre entier de 1 à 6, à condition que r + q ne dépasse pas 10;

p est 0 ou 1;

$R_3$ est un groupe alkyle en $C_{1-6}$ ou alcényle en $C_{3-6}$;

$R_9$ est OH, un groupe alkyle en $C_{1-6}$ ou alcoxy en $C_{1-6}$;

$R_{10}$ est H; $R_4CO$— ou $R_4OCH_2$—;

$R_{14}$ est un groupe alkyle en $C_{1-6}$, phényle, phényle substitué par des groupes alkyle ou alcoxy en $C_{1-3}$, halogène, hydroxy, halogénoalkyle, COOH, CN, formyle, acyle en $C_{1-6}$ ou perfluoroalkyle en $C_{1-4}$; et toutes les chaînes carbonées de tous les radicaux sont droites ou ramifiées.

2. Procédé de préparation d'un composé ayant la formule XI ou XII selon la revendication 1, dans laquelle Y' est l'oxygène et Y est l'oxygène, le soufre, un groupe sulfoxyde, sulfone, amino ou cyanamido et $R_2$ est

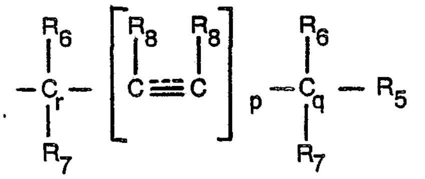 ,

dans laquelle au mouns un $R_7$ est OH, les autres variables étant telles que définies dans la revendication 1, qui comprend la condensation d'un composé de formule

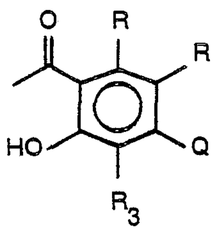 VII,

où Q est 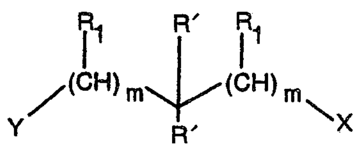 ou 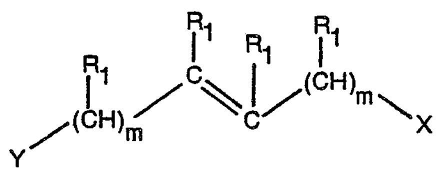

avec un composé de formule 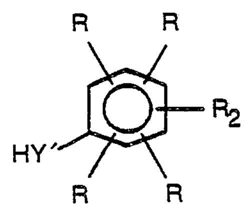

où X est un halogène.

3. Procédé de préparation d'un composé ayant la formule XI ou XII selon la revendication 1, qui comprend la condensation d'un composé ayant l'une des formules suivantes

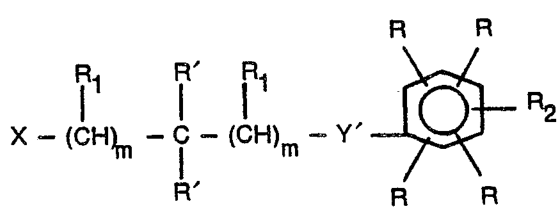

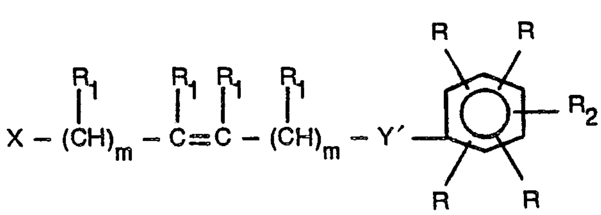

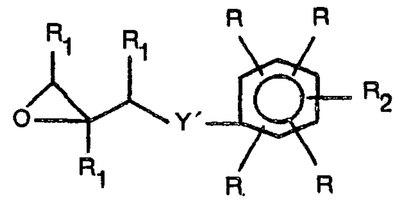

avec un composé de formule 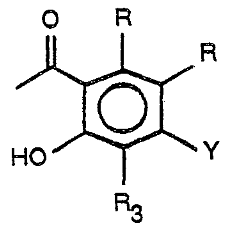 ,

dans laquelle $R_1$, $R_2$, $R_3$, R, R', Y, Y' et m sont tels que définis dans la revendication 1, et X est un halogène.

4. Procédé de préparation d'un composé ayant lf formule XI ou XII selon la revendication 1, dans laquelle Y' est l'oxygène et Y est l'oxygène, la soufre, un groupe sulfoxyde, sulfone ou cyananamido et $R_2$ est

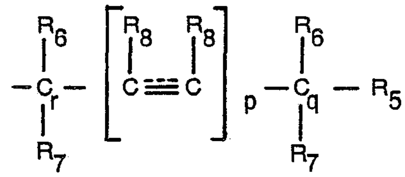 ,

dans laquelle au moins un $R_7$ est OH, les autres variables étant telles que définies dans la revendication 1, qui comprend la condensation d'un composé de formule

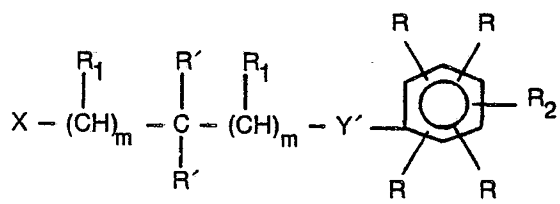

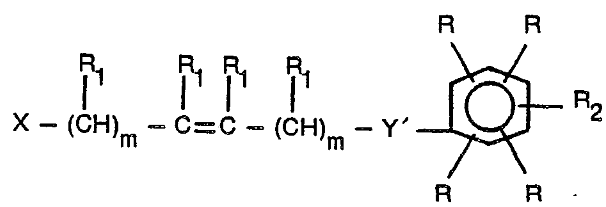

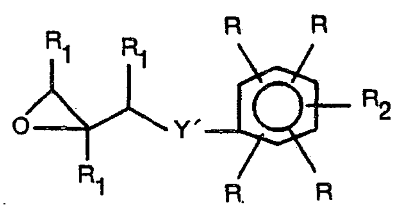

avec un composé de formule 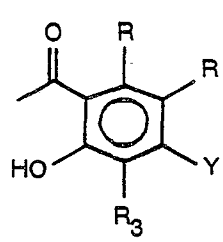 ,

où R, $R_1$, $R_2$, $R_3$, R, Y', Y et m ont la signification donnée dans la revendication 3, où X est un halogène.

5. Procédé selon la revendication 1 ou 3, s'appliquant à la préparation d'un composé dans lequel Y est l'oxygène et Y' est le soufre, un groupe sulfoxyde, sulfone, amino ou cyanamido.

6. Procédé selon la revendication 2 ou s'appliquant à la préparation d'un composé dans lequel Y' est l'oxygène et Y est l'oxygène, le soufre, un groupe sulfoxyde, sulfone ou cyanamido et $R_2$ est

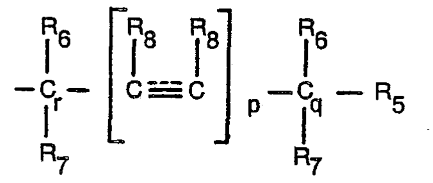 ,

dans lequel au moins un $R_7$ est OH.

7. Procédé selon la revendication 1 ou 3, s'appliquant à la préparation de l'acide 4-(3-(4-acétyl-3-hydroxy-2-propylphénoxy)propylthio)-3-fluorobenzène-acétique.

8. Procédé selon la revendication 1 ou 2, s'appliquant à la préparation de l'acide 4-(3-(4-acétyl-3-hydroxy-2-propylphénoxy)propylthio)benzène-acétique.

9. Procédé selon la revendication 1 ou 3, s'appliquant à la préparation du 4-(3-(4-acétyl-3-hydroxy-2-propylphénoxy)propylthio)-delta-hydroxybenzène-butanol.

10. Procédé selon la revendication 1 ou 3, s'appliquant à la préparation du 4-(3-(4-acétyl-3-hydroxy-2-propylphéoxy)propylsulfonyl)-delta-hydroxybenzène-butanol.